# EUROPEAN PATENT SPECIFICATION

(11) **EP 2 233 180 B1**
(45) Date of publication and mention of the grant of the patent: **10.09.2014**
(21) Application number: 10156551.3
(22) Date of filing: 15.03.2010
(51) Int. Cl.: A63B 71/06, A63B 24/00

(54) **Systems, methods, and devices for simulating real world terrain on an exercise device**
Systeme, Verfahren und Vorrichtungen zum Simulieren von echtem Gelände auf einer Trainingsvorrichtung
Systèmes, procédés et dispositifs pour simuler un terrain du monde réel dans un dispositif d'exercice

(30) Priority: 27.03.2009 US 413362; 27.03.2009 US 413330
(43) Date of publication of application: 29.09.2010
(60) Divisional application: 14170505.3
(73) Proprietor: Icon IP, Inc., Logan, UT 84321 (US)
(72) Inventor: Ashby, Darren C., Richmond, Utah 84333 (US); Watterson, Scott R., Logan Utah 84321 (US); Lorrigan, Kirk, Logan Utah 84321 (US); Dalebout, William T., Logan Utah 84341 (US)
(74) Representative: Stuttard, Garry Philip

(56) References cited:
- EP-A1- 1 683 550
- US-A1- 2005 239 601
- US-A1- 2006 122 035

## Description

### 1. The Field of the Invention

The present invention relates to exercise equipment. More specifically, the invention relates to methods, systems, and devices for selective adjustment of an exercise device to simulate movement along real world terrain.

### 2. The Relevant Technology

In an attempt to improve their health and physical conditioning, consumers are purchasing exercise devices in record quantities. One common challenge with exercise equipment is motivating the purchaser to use the device on a consistent and ongoing basis. This lack of motivation can be a result of the repetitive nature of the exercises and exercise routines that a user can perform on a specific exercise device as well as the versatility of the exercise devices.

With a typical stationary exercise cycle, for example, a user sits on a seat, holds onto one or more handles, and pedals with his or her feet. In order to provide variety during the exercise routine, the user can increase or decrease his or her pedaling rate at various times during the exercise routine. This can be done by increasing or decreasing the amount of effort the user uses to pedal or by increasing or decreasing the pedaling resistance provided by the exercise cycle. Additionally, many stationary exercise cycles are pre-programmed with one or more exercise routines that automatically adjust the pedaling resistance at various time intervals during the exercise routine. Adjusting the pedaling rate and/or the pedaling resistance can allow a user to achieve a workout suitable for the user's fitness level and goals. Adjusting the pedaling rate and/or the pedaling resistance, however, is often insufficient to maintain a user's motivation to consistently use the stationary exercise cycle.

Typical treadmills also allow a user to adjust various operating parameters to provide for improved workouts and variety during the workouts. As with the stationary exercise cycles, however, users are typically limited as to which treadmill operating parameters can be adjusted. For instance, treadmills usually provide for the adjustment of the speed and incline of the endless belt upon which the user ambulates. This allows a user to walk, jog, and/or run on the treadmill. It also allows the user to ambulate on a level surface or on an inclined surface that generally replicates a hill. These adjustable operating parameters are, like those of the stationary exercise cycles, often insufficient to motivate a user to consistently use the treadmill on an ongoing basis.

Another factor that contributes to the lack of motivation to use exercise devices is the lack of visual or other type of stimulation provided to the user while using the exercise device. In other words, users of exercise devices often become bored because their surroundings do not change during an exercise routine. Rather, their surroundings (i.e., the room in which the exercise device is located) are generally the same each time the user exercises and throughout each exercise session. This boredom can discourage the user from regularly using the exercise device. Even when the user does use the exercise device, the boredom resulting from the lack of stimulation can cause the user to not work as hard during the exercise session, which can hamper the user's ability to achieve his or her fitness goals.

In order to combat this lack of stimulation, many exercise devices are equipped with a display for providing visual stimulation and motivation to the user of the device. For example, some displays depict a tract for indicating to a user how far the user has run or pedaled. Similarly, some displays depict hills that provide a visual representation of the resistance or inclination of the device. For instance, the display of a stationary exercise cycle may depict a series of hills that are related to the pedaling resistance of the exercise cycle. As the user "rides up the hill", the pedaling resistance will increase: the steeper the hill, the greater the pedaling resistance will be. Correspondingly, as the user "rides down the hill", the pedaling resistance will decrease. While these types of displays may provide some visual stimulation to the user, most users will quickly become bored with such displays, and the desired stimulatory benefits will not be realized. In contrast, when a person goes outside for a walk, run or bicycle ride, the person's surroundings are constantly changing, which can provide sufficient stimulation to the person's mind to keep them motivated throughout the exercise routine.

US 2005/0239601 is concerned with a virtual exercise system and method. A virtual racecourse is mapped by uniting digital pictures with GPS and inclinometer data which is stored in a recorded course file and accessed for individual and group competition over the internet.

### BRIEF SUMMARY OF THE INVENTION

It is an object of the present invention to provide an exercise system and a method for generating exercise programming. This object can be achieved by the features of the independent claim. Further embodiments are characterized by the dependent claims.

The present invention relates to exercise equipment and systems, and particularly to methods, systems, and devices for selective adjustment of an exercise device to simulate movement along real world remote locations while displaying images/videos of the remote locations. Simulation of a remote, real world locations and display of the related images provides a user of the exercise device with the greater interest and motivation to use the exercise device on a regular and ongoing basis. The simulation also allows the user to experience is a very real sense what it is like to traverse the remote location.

Thus, according to the invention as claimed in claim 1, an exercise system includes one or more exercise devices that communicate via a network with a communication system, such as a website. The communication system stores and/or generates exercise programming for use on the exercise device. The exercise programming is able to control one or more operating parameters of the exercise device to simulate terrain found at a remote, real world location. The exercise programming can also include images/videos of the remote, real world location. The control signals and the images/videos can be synchronized so that a user of the exercise device is able to experience, via the changing operating parameters, the topographical characteristics of the remote, real world location as well as see images of the location.

In the present invention, the exercise system includes an exercise device that has a movable element for movement in performance of exercise by a user and at least one actuator for controlling one or more operating parameters of the exercise device. The exercise device is able to receive exercising programming that causes the exercise device to simulate one or more aspects of a remote, real world exercise route. The exercise programming can include one or more control signals representative of changes to be made to the one or more operating parameters to simulate the remote, real world exercise route. In some embodiments, the exercise programming includes display programming that display images of the remote, real world exercise route to the user. The control signals and the display programming can be synchronized with one another to enhance the simulated experience.

The exercise system also includes a remote communication system that uses data relating to the remote, real world exercise route to generate the exercise programming which will cause the exercise device to simulate the one or more aspects of the remote, real world exercise route. The data used to generate the exercise programming can include map data, topographical data, video or image data, or a combination thereof. The data used to generate the exercise programming can be obtained from one or more databases, such as one or more websites. One database, for example, may store topographical data while another database stores image data. The remote communication system can be configured to access the data from one or more of the databases and synchronize the data to generate the exercise programming. The system also includes a network adapted to facilitate communication between the remote communication system and the databases as well as facilitating communication of the exercise programming from the remote communication system to the exercise device.

The exercise system includes an exercise device that has a movable element for movement in performance of exercise by a user. The exercise device can also have one or more operating parameters that are controlled by exercise programming. A remote communication system can be adapted to communicate with a user of the exercise device and generate the exercise programming that is communicated to the exercise device. The remote communication system enables the user to create user defined exercise programming by allowing the user to select a starting point and an ending point for the remote, real world exercise route. As above, the exercise programming can include one or more control signals representative of changes to be made to the one or more operating parameters to simulate topographical characteristics of the remote, real world exercise route, and display programming including images of the remote, real world exercise route. The exercise system can also include a network that communicates with the exercise device and the remote communication system to communicate the exercise programming from the remote communication system to the exercise device.

In one embodiment not covered by the claims, the exercise device is a treadmill that has a base frame, a treadbase mounted on the base frame, and an endless belt trained around the treadbase to enable the user to ambulate thereon. The treadbase can be pivotally mounted on the base frame so that the treadbase can be selectively inclined or declined to simulate for the user the experience of ambulating up and down hills of a remote, real world exercise route. Similarly, the treadbase can be pivotally mounted on the base frame so that the treadbase can be selectively tilted from one side to the other side to simulate for the user the experience of ambulating on an uneven surface of a remote, real world exercise route.

In another embodiment not covered by the claims, the exercise device is an exercise cycle. The exercise cycle includes a support base adapted to rest upon a support surface and an upright support structure mounted to the support base. The upright support structure includes a seat, a handle bar assembly, and a control panel. The exercise cycle also includes a pedal assembly that can be engaged and rotated by a user's feet. A resistance assembly provides resistance to the rotation of the pedal assembly and can be controlled by one or more control signals of the exercise programming. According to some embodiments, the upright support structure is pivotally mounted to the support base so that the upright support structure can be selectively tilted forward or backward to simulate for the user the experience of riding a bicycle up or down a hill.

These and other objects and features of the present invention will become more fully apparent from the following description and appended claims, or may be learned by the practice of the invention as set forth hereinafter.

### BRIEF DESCRIPTION OF THE DRAWINGS

To further clarify the above and other advantages and features of the present invention, a more particular description of the invention will be rendered by reference to specific embodiments thereof which are illustrated in the appended drawings. It is appreciated that these drawings depict only illustrated embodiments of the invention and are therefore not to be considered limiting of its scope. The invention will be described and explained with additional specificity and detail through the use of the accompanying drawings in which:

Figure 1 illustrates an exemplary exercise system according to the present invention;

Figure 2 is a perspective illustration of a treadmill to be used in the exercise system of Figure 1;

Figure 3 is a side illustration of a the treadmill of Figure 2 with the treadbase shown in a neutral position, and an inclined position featured in phantom view;

Figure 4 is a rear perspective illustration of the treadmill of Figure 2 with the treadbase in a first tilted position;

Figure 5 is another rear perspective illustration of the treadmill of Figure 2 with the treadbase in a second tilted position;

Figure 6 is a bottom view of the treadmill illustrated in Figures 2 through 5 showing some of the incline/decline and tilting mechanisms of the treadmill;

Figure 7 is a cut away perspective view of the incline mechanism incorporated into the treadmill illustrated in Figures 2 through 6;

Figure 8 is a cut away perspective view of the tilt mechanism incorporated into the treadmill illustrated in Figures 2 through 6;

Figure 9 is a cut away perspective view of a bracket assembly incorporated into the treadmill illustrated in Figures 2 through 6 which facilitates inclination/declination and tilting of the treadbase relative to the base frame;

Figure 10 is a perspective illustration of a stationary exercise cycle to be used in the exercise system of Figure 1;

Figure 11 is a side illustration of the stationary exercise cycle of Figure 10 with the upright frame shown in a forward tilted position, and a neutral position featured in phantom view;

Figure 12 is another side illustration of the stationary exercise cycle of Figure 10 with the upright frame shown in a backward tilted position, and a neutral position featured in phantom view;

Figure 13 is a perspective illustration of the control panel of the treadmill of Figure 2;

Figure 14 is a functional block diagram of the process of connecting to a remote communication system and selecting one or more options available from the remote communication system;

Figure 15 is a flow diagram representing the actions performed by the user and communication module to create exercise programs in accordance with the teachings of the present invention;

Figure 16 is a functional block diagram of the process of selecting an exercise device to be used in connection with the exercise programs; and

Figure 17 is a flow diagram of the process of accessing a remote communication system, creating exercise programming, and downloading the exercise programming for use an on exercise device.

### DETAILED DESCRIPTION OF THE PREFERRED EMBODIMENTS

The present invention is directed to devices that include one or more motors or other electrically driven actuators used to control one or more operating parameters of the device. While the invention will be described in the context of either a motorized treadmill or a stationary exercise cycle, it should be understood that the invention is not limited to any particular type of exercise device. To the contrary, the present invention can be readily adapted to any motorized device or any other device that utilizes motors, solenoids, or any other electrically driven actuators to control any operating parameter of the device, such as speed, resistance, incline, time, temperature, or other similar operating parameters. The term "device" or "devices" shall refer broadly to any type of apparatus that includes one or more stepper motors, solenoids, or other electrically driven actuators or controllers. Additionally, the term "exercise devices" shall refer broadly to any type of device that takes the form of an exercise machine, including, but not limited to, treadmills, exercise cycles, Nordic style ski exercise devices, rowers, steppers, hikers, climbers, and elliptical or striding exercise devices.

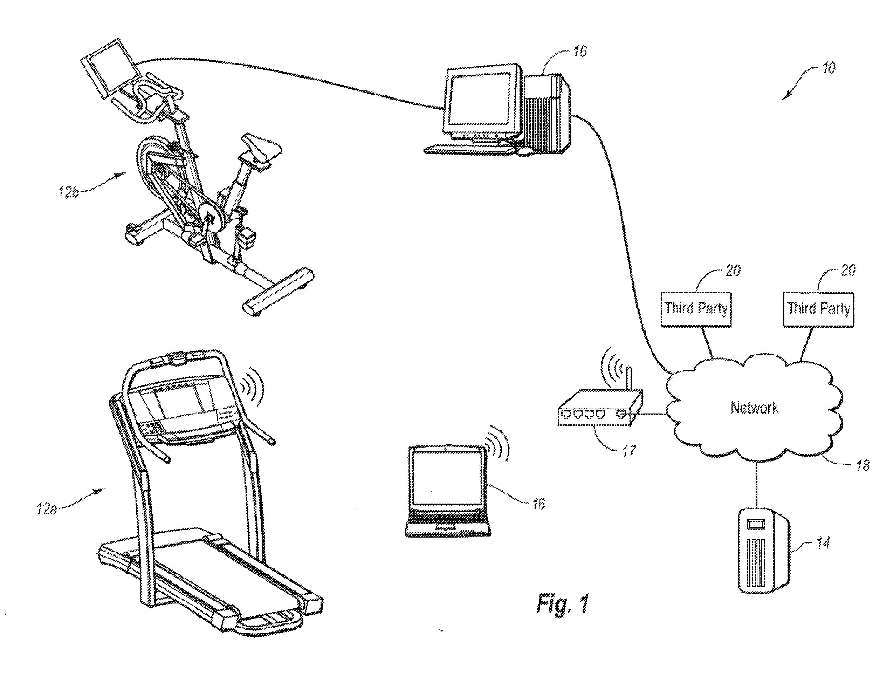

Depicted in Figure 1 is a representation of one illustrative system, designated by reference numeral 10, which may incorporate the novel features of the present invention, including various novel devices, hardware and software modules, and the like. As shown, one or more exercise mechanisms 12, such as a treadmill 12a and an exercise cycle 12b are each in communication with a communication system 14 (e.g. a website) via a personal computer 16. The personal computers 16 communicate with a network 18 that is a communication network that enables various hardware and software modules and devices to communicate one with another. Network 18, therefore, may be a local area network (LAN), wide area network (WAN), wireless network, packetized network, real-time network, and the like. Network 18 facilitates communication of treadmill 12a and/or exercise cycle 12b with communication system 14. Communication system 14 assists with communication between a user on treadmill 12a and/or exercise cycle 12b and one or more third parties 20, as will be described in more detail hereinafter.

In the illustrated embodiment, connection between the exercise mechanisms 12 and network 18 can be made via a variety of communication line connections. For example, as depicted in Figure 1, treadmill 12a is capable of wireless communication with network 18, either directly or via computer 16 and/or wireless router 17. Various other types of ports or interfaces may be included within exercise mechanisms 12 to enable communication via one or more communication line connections. For instance, an exercise mechanism 12 may include one or more ports and interfaces to enable communication line connection through existing broadcast technology, including television broadcast over the airwaves, cable or cable modems, satellite, telephone lines, whether analog or digitally based, the Internet, DSL, G-Lite, wireless technology, infrared (IR) technology, other high-speed data connections, or any other suitable transmission technology or medium. In the illustrated embodiment, exercise cycle 12b is shown with a hardwire connection to personal computer 16, which has a hardwire connection with network 18. Thus, system 10 may allow for any type of connection between an exercise mechanism 12 and network 14, whether wired or wireless.

Although each exercise mechanism of system 10 is depicted as communicating via a personal computer 16 and network 18 with a single communication system 14 and two third parties 20, it may be appreciated by one skilled in the art that system 10 may be otherwise configured. For example, treadmill 12a and exercise cycle 12b may communicate via the same personal computer 16. Similarly, the exercise devices may communicate with multiple communications systems 14 and third parties 20 via multiple networks 18. Alternatively, one or more of the elements of system 10 may be eliminated or the functionality thereof incorporated within the structure and function of one or more of the other elements of system 10.

Similarly, although each of the elements of system 10 are shown separated one from another, it may be appreciated by one skilled in the art that the hardware and/or software elements of the present invention may be incorporated within two or more elements. For example, personal computers 16 may be incorporated within treadmill 12a and/or exercise cycle 12b. Similarly, the hardware and/or software elements of the communication system 14 may be incorporated within treadmill 12a and/or exercise cycle 12b.

As used herein, a database that is "external to" or "remote from" communication system 14 refers to a database that is administered or maintained by a third party 20 that is different than the entity that administers or maintains communication system 14. Generally, examples of a third party 20 may include: (i) a live human being; or (ii) a database, such as a website, computer, optical media (e.g., compact disk or digital video disk), visual media, or magnetic media (e.g., videotape, readable disk), an electronic monitoring system, dynamic computer readable instructions, interactive and/or dynamic software programs, computer readable instructions, one or more other databases, other media, hardware, and/or software modules and components that is/are located external to communication system 14. In some embodiments, a third party 20 may include MAPQUEST.COM, MAP.GOOGLE.COM, the GOOGLE EARTH database, the GTOPO 30 database, the GOOGLE STREET VIEW database, the MICROSOFT VIRTUAL EARTH database, and the like. These third parties are examples of databases that store data external to communication system 14.

Such databases store image data that can be displayed or can be formatted or manipulated to be displayed on a display device 152 of an exercise device 12. The term "image data" includes and/or is representative of: i) one or more static images; and/or ii) one or more moving (i.e., video) images. For example, image data as used herein may include a plurality of sequential static images, a video display, and/or a single image of terrain to be traversed by a user, such as a mountain, race course, or street.

Furthermore, the phrase "display programming," as used herein, includes image data and/or image data that has been formatted or manipulated so that it can be synchronized with control signals and/or displayed on a display device of an exercise device. Examples of such display programming that can display images on display 152 include video programming, sequential static image programming, and/or a single image of terrain to be traversed, for example.

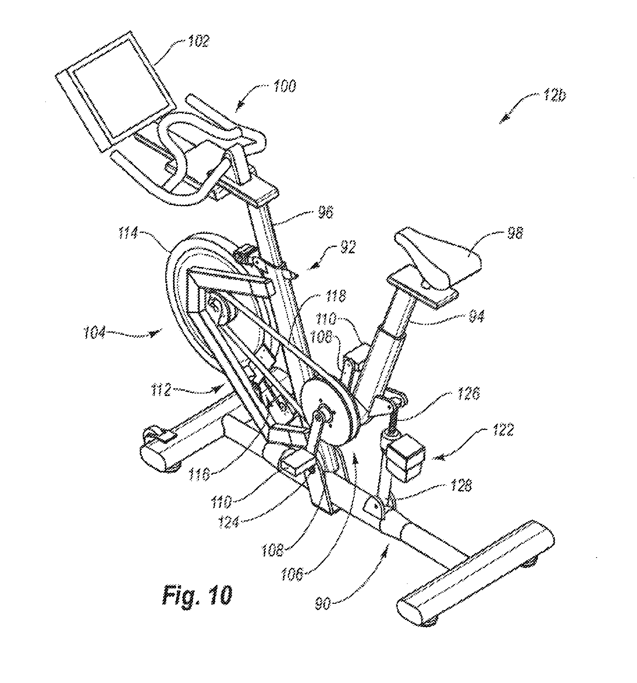

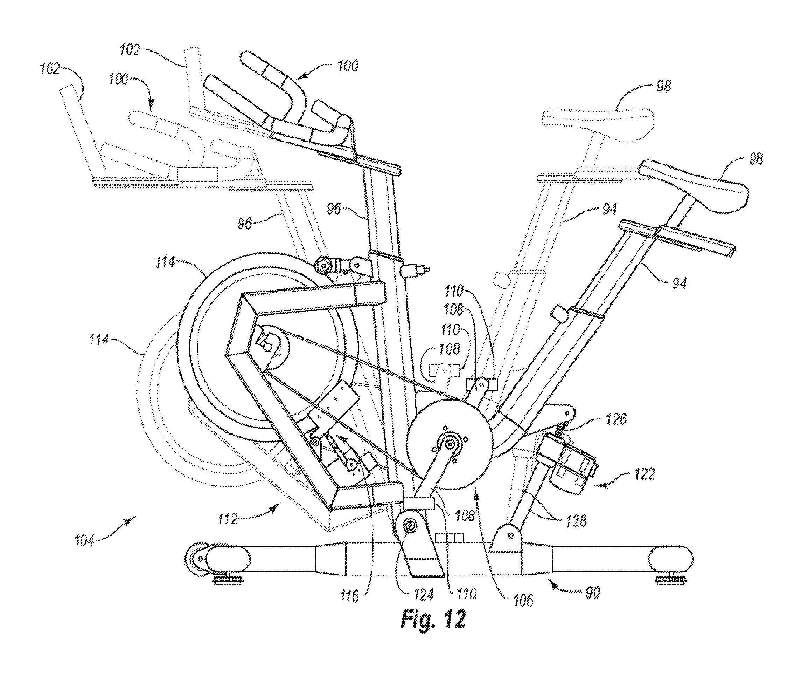

The majority of the discussion of system 10 will focus on the use and interaction of treadmill 12a with system 10. However, exercise cycle 12b will also be described in connection with Figures 10 through 12. While system 10 will be described primarily in connection with treadmill 12a, it may be appreciated that a similar discussion may be had for exercise cycle 12b, other types of exercise mechanisms, or multiple exercise mechanisms of the same or different type. Thus, as illustrated in Figure 1, exercise cycle 12b may be used in connection with system 10 along with most of the features described in connection with treadmill 12a.

Generally, system 10 enables exercise programming with control signals to be transmitted from communication system 14, to a user at treadmill 12a. The programming may include motivational content and/or one or more control signals that may be used to control the operating parameters of treadmill 12a. The control signals may be synchronized with the motivational content and designed to control one or more operating parameters of the exercise device, such as the speed, incline, difficulty of exercise program, time, distance, and the like of an exercise program performed on treadmill 12a.

As used herein, the term "motivational content" is used to broadly refer to any video or visual material either alone or in combination with audio material, including dialog, narration, sound effects, and/or music. In one embodiment of the present invention, the motivational content is stored by a third party 20 and includes images, whether still or moving, of real world environments, routes, locations, and the like.

Various terms are used herein to describe actual outdoor exercise experiences that can be simulated on treadmill 12a, or another exercise device. These terms include real world environments, places, routes, trails, paths, courses, hikes, locations, and the like. It will be appreciated that these terms are used to broadly refer to characteristics of actual places in the world, including the topography, appearance, and sounds associated with the real world places. Additionally, exercise system 10 is described as being able to simulate these real world places. Simulating these real world places refers to providing a user of an exercise device an experience that is similar to actually being in the real world places. In other words, system 10 is adapted to replicate on an exercise device the topography, sights, and/or sounds that a person would experience were the person to actually to walk, run, ride, or the like, through the actual real world location.

Generally, communication between treadmill 12a and communication system 14 and/or a third party 20 may include both the motivational content and the control signals, whether or not such control signals are synchronized with the motivational content. Alternatively, the communication may include only the motivational content, other signals representative of measurable parameters of the exercise device (e.g. speed, inclination, resistance, etc) and/or a user of the exercise device (e.g. heart rate, blood pressure, etc), and the like. For example, treadmill 12a may transmit one or more signals to communication system 18. The signal may include parameters such as the status of the exercise device, e.g., active status (i.e., on), deactivated status (i.e., off), standby status (i.e., waiting), and the like, and/or parameters such as speed, inclination, resistance. Additionally, the signal may include parameters regarding the user, such as heart rate, blood pressure, and the like. Alternatively, treadmill 12a may receive programming "broadcast" by communication system 14, such that any treadmill with the capabilities to receive the programming may access such, without the need to transmit one or more signals.

As mentioned above, the control signals control the operating parameters of treadmill 12a, such as speed, inclination, resistance, and the like. Such control may be achieved by communication system 14, or a combination of communication system 14 and a third party 20 interacting with treadmill 12a and/or communication system 14. Generally, the present invention allows control of a device, such as an exercise device, without the need to interrupt the other portions of the programming, such as the real-time audio and/or video.

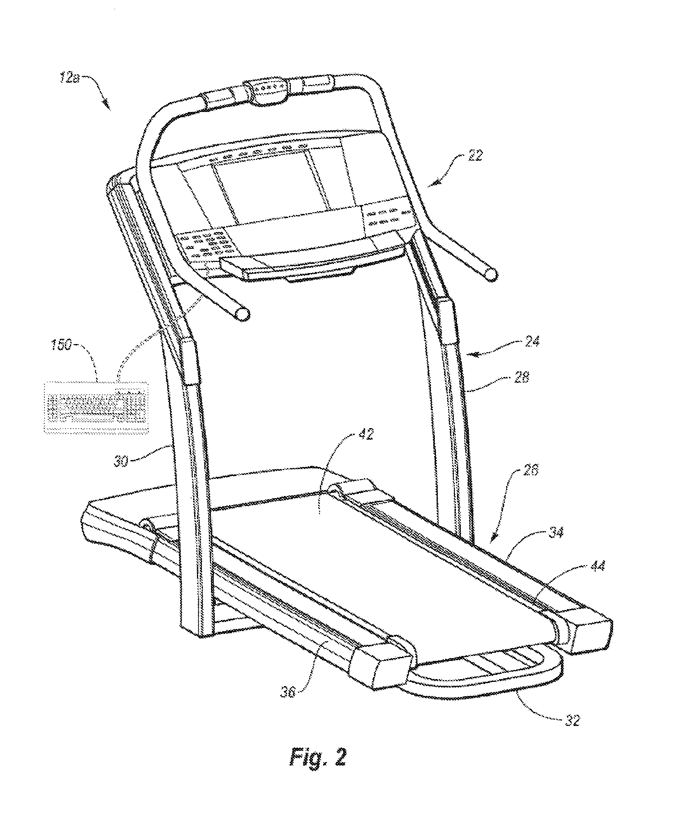

Figures 2 through 9 generally depict a typical motorized treadmill 12a that can be used in connection with system 10. Treadmill 12a, in one embodiment, includes a control panel 22 supported on a generally upright support structure 24 and a treadbase 26. Upright support structure 24, in this illustrative embodiment, includes two side members 28, 30 coupled to a base frame 32. Side members 28, 30 and base frame 32 may have various configurations and may be fabricated from various materials so long as they are capable of supporting control panel 22 and treadbase 26. For example, the elements of upright support structure 24 and base frame 32 may be fabricated from, but not limited to metals, plastics, composites, combinations thereof, and the like. Additionally, one skilled in the art may appreciate that various other exercise devices may have different upright support structures, side members, and base frames, or be devoid of one or more of such structures and members.

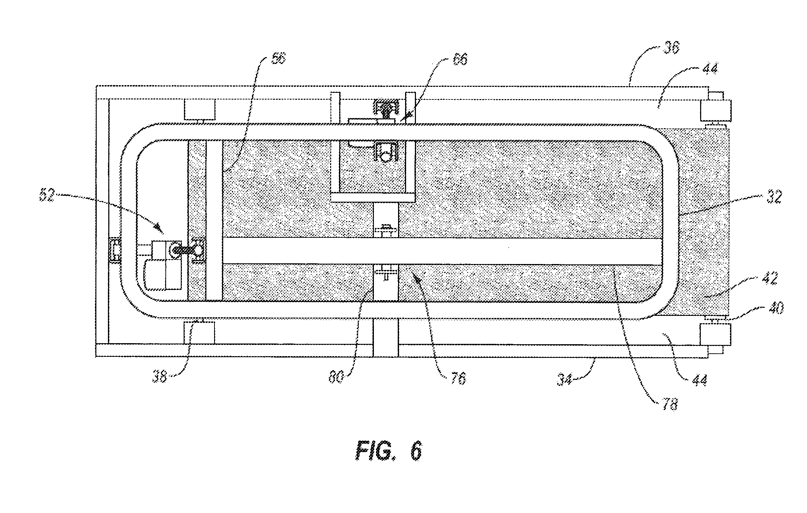

The treadbase 26 typically includes a pair of side rails 34, 36 each having a front portion proximal to and a rear portion distal from upright support structure 24. A front pulley 38 (Figure 6) and a rear pulley 40 (Figure 6) are disposed between and supported by side rails 34, 36, while a continuous belt 42 extends between and around front and rear pulleys 38 and 40, respectively. Pulleys 38, 40 and belt 42 may have various configurations and be fabricated from various materials, as known by one skilled in the art and commonly known within the exercise industry.

A deck 44, commonly fabricated from wood, typically supports the upper run of belt 42 and supports an exercising individual positioned upon belt 42. Although deck 44 is preferably of a cellulose material such as wood, various other types of material may be used so long as deck 44 is capable of supporting belt 42 and a user exercising thereupon.

As is common with electric treadmills, such as treadmill 12a, front pulley 38 is mechanically coupled to an electric tread drive motor 46 (not shown) by way of a drive belt 48 (not shown). Motor 46 can incorporate an inertial flywheel that controls fluctuations in the rotational motion of a shaft of motor 46 during operation of treadmill 12a. Motor 46 is optionally electrically coupled to a treadmill controller 50 (not shown) that controls the operation of motor 46, and thus the speed of belt 42, in response to various user inputs or other control signals. Treadmill controller 50 can be incorporated within treadbase 26, control panel 22, or within personal computer 16.

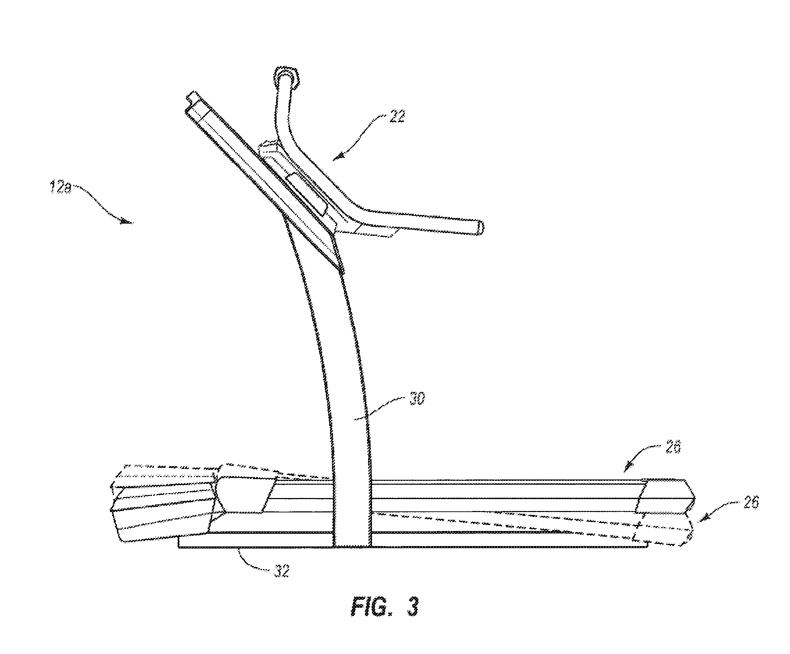

In addition to the ability to control and vary the speed of belt 42, treadmill 12a also permits the degree of incline of treadbase 26 relative to the floor, or other surface upon which treadbase 26 rests, to be varied. As depicted in Figure 3 in solid lines, treadbase 26 can be oriented in a neutral position. In the neutral position, treadbase 26 is substantially parallel to a support surface. Additionally, as illustrated in phantom lines in Figure 3, treadbase 26 can be oriented in an inclined position such that the front portion of treadbase 26 is above the neutral position. This enables an exerciser to simulate walking or running up a hill.

In one embodiment, treadbase 26 can also be configured to decline into a declined position in which the front portion of treadbase 26 drops below the neutral position. Typical walks or runs outside, for example, involve inclines and declines as well as flat surfaces, each of which can be accommodated and replicated by treadbase 26. Thus, treadmill 12a is able to more closely simulate typical outdoor terrain.

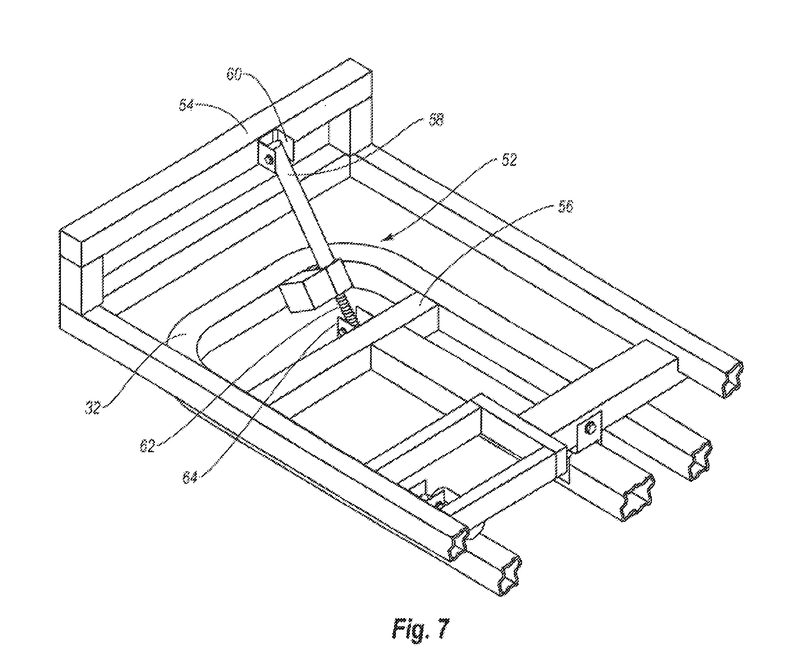

The inclination/declination of treadbase 26 can be accomplished through the use of an incline mechanism 52, as depicted in Figures 6 and 7, or another linearly extending assembly. Incline mechanism 52 raises or lowers one end of treadbase 26 relative to the other end. In the embodiment illustrated in Figures 6 and 7, incline mechanism 52 is pivotally coupled to a cross bar 54 of treadbase 26 and a cross bar 56 of base frame 32.

More particularly, a first end 58 of incline mechanism 52 is pivotally coupled to cross bar 54 by way of a bracket 60 while a second end 62 of incline mechanism 52 is coupled to cross bar 56 by way of a bracket 64. Each of brackets 60, 64 are generally U-shaped with a pin that can be secured between the two extending sides. The first and second ends 58, 62 of incline mechanism 52 are each mounted on the pin of their respective bracket 60, 64. As incline mechanism 52 raises or lowers the end of treadbase 26, first and second ends 58, 62 of incline mechanism 52 are able to pivot on the pins within brackets 60, 64. In this manner, first and second ends 58, 62 of incline mechanism 52 are able to pivot about axes that are generally transverse to a longitudinal axis of treadbase 26. As will be discussed in greater detail below, brackets 60, 64 can be pivotally coupled to cross bars 54, 56 to allow treadbase 26 to tilt from side to side without damaging incline mechanism 52.

In one embodiment, upon contraction of incline mechanism 52, treadbase 26 moves to a declined position such that the front end of treadbase 26 is positioned below the neutral position. When incline mechanism 52 is selectively extended to an extended position, treadbase 26 is inclined such that the front end of treadbase 26 is positioned above the neutral position. Through the inclination/declination of treadbase 26, as described above, treadmill 12a is able to simulate for a user the experience of walking or running on level ground, up hills, and down hills.

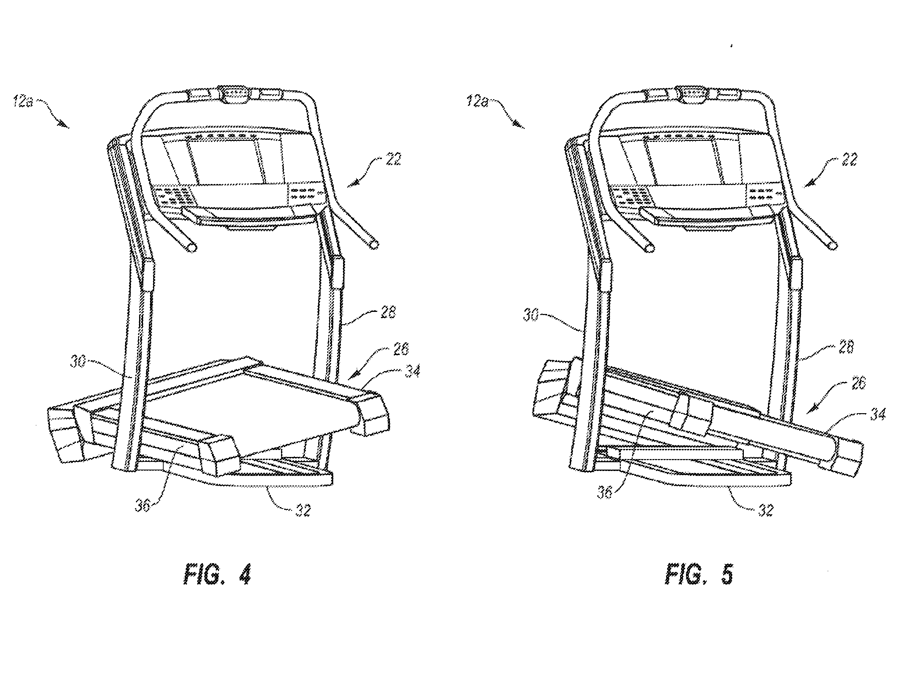

In the illustrated embodiment, treadmill 12a also permits treadbase 26 to be tilted from side to side in order to more closely replicate walking or running on outdoor terrain. As depicted in Figures 4 and 5, treadbase 26 can be tilted such that one side of treadbase 26 is higher than the other. For instance, Figure 4 illustrates treadbase 26 tilted so that side rail 34 is higher than side rail 36. Similarly, Figure 5 illustrates treadbase 26 tilted so that side rail 36 is higher than side rail 34. The ability to tilt treadbase 26 to one side or another allows treadmill 12a to more closely simulate outdoor terrain. In particular, when a person goes outside for a run or walk, the person often encounters both inclining and declining hills as well as surfaces that are not level from side to side, such as when walking or running across a hill. Thus, treadmill 12a more closely replicates an outdoor walking or running experience by providing a surface that inclines, declines, and tilts to each side.

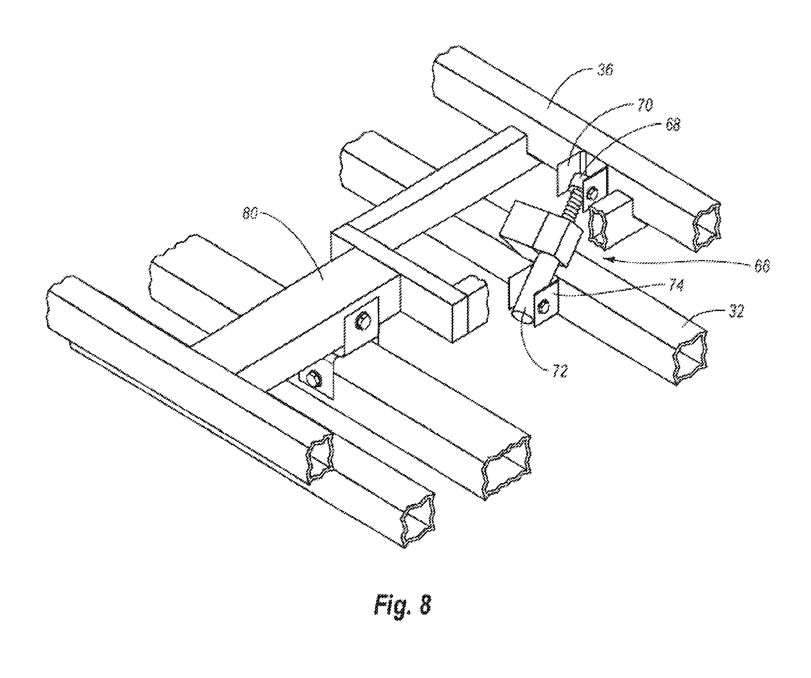

The tilting of treadbase 26 can be accomplished through the use of a tilt mechanism 66, as depicted in Figures 6 and 8, or another linearly extending assembly. Tilt mechanism 66 is substantially similar to incline mechanism 52. Tilt mechanism 66 raises or lowers one side of treadbase 26 relative to the other side. In the embodiment illustrated in Figures 6 and 8, tilt mechanism 66 is pivotally coupled to side rail 36 of treadbase 26 and base frame 32.

More particularly, a first end 68 of tilt mechanism 66 is pivotally coupled to side rail 36 by way of a bracket 70 while a second end 72 of tilt mechanism 66 is coupled to base frame 32 by way of a bracket 74. Each of brackets 70 and 74 are generally U-shaped with a pin that can be secured between the two extending sides. The first and second ends 68, 72 of tilt mechanism 66 are each mounted on the pin of their respective bracket 70, 74. As tilt mechanism 66 raises or lowers the side of treadbase 26, first and second ends 68, 72 of tilt mechanism 66 are able to pivot on the pins within brackets 70, 74. In this manner, first and second ends 68, 72 of tilt mechanism 66 are able to pivot about axes that are generally parallel to a longitudinal axis of treadbase 26. As will be discussed below, brackets 70, 74 can be pivotally coupled to side rail 36 and base frame 32 to allow one end of treadbase 26 to incline relative to the other without damaging tilt mechanism 66.

In one embodiment, upon contraction of tilt mechanism 66, side rail 36 is moved to a lower position than side rail 34. When tilt mechanism 66 is selectively extended to an extended position, side rail 36 is moved to a higher position than side rail 34. Through the tilting of treadbase 26, as described above, treadmill 12a is able to more closely simulate for a user the experience of walking or running outdoors.

As noted above, brackets 60, 64, 70, 74 can be pivotally coupled to their respective cross bars or frames. Pivotally coupling brackets 60, 64, 70, 74 to their respective cross bars or frames prevents incline mechanism 52 and tilt mechanism 66 from being bent or otherwise damaged when treadbase 26 is inclined, declined, or tilted. For instance, the pivoting connection between brackets 70, 74, side rail 36, and base frame 32 allows treadbase 26 to incline or decline without bending, overextending, or otherwise damaging tilt mechanism 66. Similarly, the pivoting connection between brackets 60, 64 and cross bars 54, 56 allows treadbase 26 to tilt without bending, overextending, or otherwise damaging incline mechanism 52.

Brackets 60, 64, 70, 74 can be pivotally coupled to their respective cross bars or frames with the use of a mechanical fastener, such as a bolt. The bottom cross portion of the generally U-shaped bracket can have a bolt extending therethrough and which is secured within the respective cross bar or frame of each bracket. The bolt connection can allow the bracket to rotate thereon. It will be appreciated, however, that one or more of brackets 60, 64, 70, 74 can be connected to their respective cross bars or frames is a non-pivoting or non-rotating manner. For example, one or more of brackets 60, 64, 70, 74 can be welded to their respective cross bars or frames.

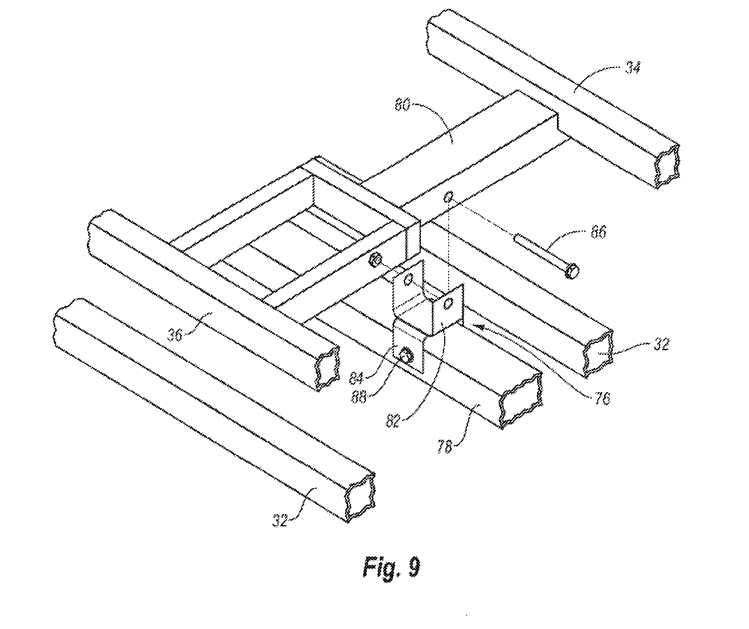

With continuing attention to Figure 6, attention is now also directed to Figure 9, which illustrates a bracket assembly 76. Bracket assembly 76 facilitates the inclination, declination, and tilting of treadbase 26 relative to base frame 32. As best seen in Figure 6, base frame 32 includes a cross member 78 that extends between the rear end of base from 32 and cross member 56. Cross member 78 is generally in line with incline mechanism 52. Treadbase 26 also includes a cross member 80 that extends between side rails 34 and 36. Cross member 80 is generally in line with tilt mechanism 66. As can be seen in the Figures, cross member 80 extends across and over cross member 78 such that cross members 78 and 80 are generally perpendicular to one another.

Bracket assembly 76 pivotally couples together treadbase 26 and base frame 32. In the illustrated embodiment, bracket assembly 76 comprises two generally U-shaped brackets 82, 84. Each of brackets 82, 84 have two substantially parallel walls and a cross member that connects the walls, thereby forming a channel within each bracket 82, 84. The cross members of bracket 82, 84 are coupled together such that the walls of bracket 82 extend in an opposite direction of the walls of bracket 84. As best seen in Figure 9, brackets 82, 84 are coupled together such that the channels formed by brackets 82, 84 extend in generally perpendicular directions. In other words, brackets 82, 84 are offset by about 90°. Brackets 82, 84 can be coupled together by any suitable means, including welding, bolts, and the like.

Cross member 80 is pivotally coupled within the channel of bracket 82 with a pin 86. Pin 86 extends through the walls of bracket 82 and through a hole in cross member 80. Coupling cross member 80 to bracket 82 in this manner enables treadbase 26 to tilt relative to bracket assembly 76 about an axis that is substantially parallel to a longitudinal axis of treadbase 26.

Bracket assembly 76 is coupled to base frame 32 is a similar manner as treadbase 26. Specifically, cross member 78 is pivotally coupled within the channel of bracket 84 with a pin 88. Pin 88 extends through the walls of bracket 84 and through a hole in cross member 78. Coupling cross member 78 to bracket 84 in this manner enables bracket assembly 76 and treadbase 26 to pivot relative to base frame 32 about an axis that is substantially perpendicular to a longitudinal axis of treadbase 26. Pivoting of bracket assembly 76 and treadbase 26 in this manner allows treadbase 26 to be inclined or declined as described herein.

Attention is now directed to Figures 10 through 12, which generally illustrate an exercise cycle 12b that can be used with system 10. Exercise cycle 12b, in one embodiment, includes a support base 90 and a generally upright support structure 92 pivotally coupled thereto. Upright support structure 92, in this illustrative embodiment, includes two support members 94, 96. Support member 94 includes a seat 98 upon which a user may sit when exercising on exercise cycle 12b. Support member 96 includes a handlebar assembly 100 and a control panel 102.

In the illustrative embodiment, a drive assembly 104 is mounted on upright support structure 92. Drive assembly 104 includes a rotatable pedal assembly 106. Pedal assembly 106 includes a pair of cranks 108 that are rotatably mounted on support member 94. Attached to each crank 108 is a pedal 110, which a user can engage with their feet to rotate pedal assembly 106. As will be appreciated by one skilled in the art, pedal assembly 106 can also be mounted on support member 96 or support base 90.

Drive assembly 104 also includes a resistance assembly 112 for providing resistance to the rotation of pedal assembly 106. Resistance assembly 112 includes a flywheel 114 and a braking mechanism 116 mounted on support member 96. Braking mechanism 116 is adapted to selectively adjust the rotational speed of flywheel 114. Resistance assembly 112 is coupled to pedal assembly 106 by an endless belt or chain 118 such that the rotational speed of pedal assembly 106 and flywheel 114 are related to one another.

Braking mechanism 116 can comprise a frictional brake, a magnetic brake, or any other suitable brake for controlling the rotational speed of flywheel 114. Braking mechanism 116 is optionally coupled to an exercise cycle controller 120 (not shown), which is similar to treadmill controller 50. Exercise cycle controller 120 controls the operation of braking mechanism 116, and thus the rotational speed of flywheel 114 in response to various user inputs or other control signals. Exercise cycle controller 120 can be incorporated within resistance assembly 112, control panel 102, or within personal computer 16.

Because resistance assembly 112 is coupled to pedal assembly 106, the braking provided to flywheel 114 by braking mechanism 116 affects the resistance to the rotation of pedal assembly 106. In other words, when a large braking force is applied to flywheel 114, it is harder for a user to rotate pedal assembly 106. Conversely, when little or no braking force is applied to flywheel 114, it is relatively easy for a user to rotate pedal assembly 106. By adjusting the amount of braking applied to flywheel 114, exercise cycle 12b can thus vary speed at which a user can pedal and/or the resistance experienced by the user as he or she pedals on exercise cycle 12b. In this manner exercise cycle 12b is able to simulate the types of resistances and pedaling speeds that a user may experience if riding a bicycle outdoors.

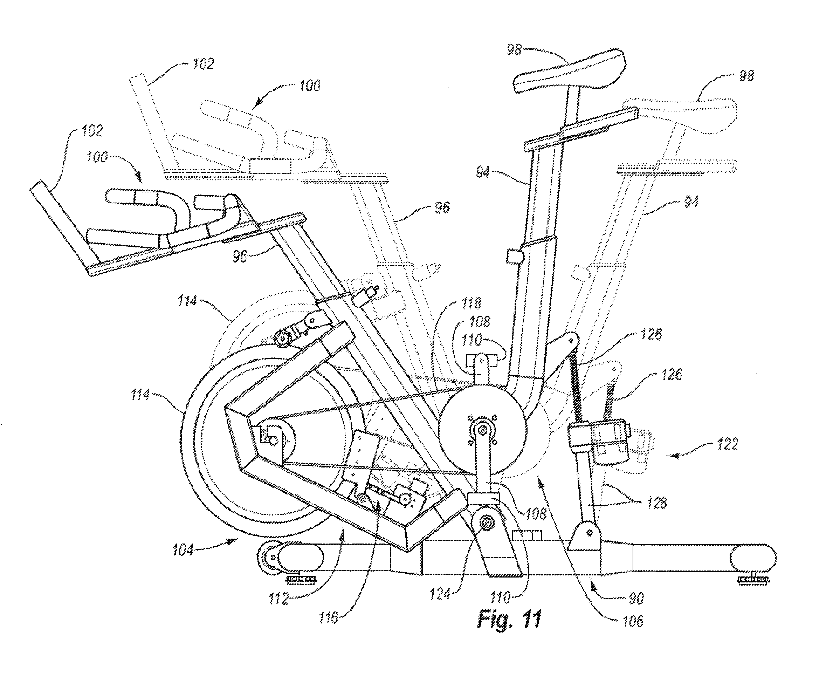

In addition to the ability to control and vary the speed and resistance of pedal assembly 106, exercise cycle 12b also permits the tilting of upright support structure 92 relative to the floor, or other surface upon which exercise cycle 12b rests, to be varied. As depicted in Figure 11 in phantom lines, upright support structure 92 can be oriented in a neutral position. In the neutral position, handle bar assembly 100 and seat 98 are at generally the same vertical height. When upright support structure 92 is in the neutral position, a user sitting on seat 98 will feel that he or she is sitting on a bicycle that is on a level surface. Additionally, as illustrated in solid lines in Figure 11, upright support structure 92 can be oriented in a forwardly tilted position such that the handle bar assembly 100 is vertically below the neutral position and seat 98. Tilting upright support structure 92 forward as illustrated in Figure 11 enables a user to simulate riding down a hill.

In one embodiment, upright support structure 92 can also be oriented in a backwardly tilted position in which the handle bar assembly 100 is vertically above the neutral position and seat 98. Typical bicycle rides outside, for example, involve inclines and declines as well as flat surfaces, each of which can be accommodated and replicated by the tilting ability of upright support structure 92. Thus, exercise cycle 12b is able to more closely simulate a typical outdoor bicycle ride.

The forward and backward tilting of upright support structure 92 can be accomplished through pivotally coupling upright support structure 92 to support base 90 as depicted in Figures 10 through 12. As seen in the Figures, upright support structure 92 is connected to support base 90 by pivot 124. Pivot 124 allows upright support structure 92 to tilt forward and backward as described above. Pivot 124 can include a pin that extends through a portion of support base 90 and through upright support structure 92. Pivot 124 can also include one or more stops to limit the tilting of upright support structure 92 within a desired range.

While pivot 124 allows upright support structure 92 to tilt forward and backward, extension mechanism 122, or another linearly extending assembly, controls the tilting of upright support structure 92. In the illustrative embodiment, extension mechanism 122 is coupled between support base 90 and support member 94. More particularly, a first end 126 of extension mechanism 122 pivotally couples to support member 94 while a second end 128 of extension mechanism 122 pivotally couples to support base 90. Extension mechanism 122 raises or lowers support member 94 relative to support base 90, thereby determining the tilt of upright support structure 92. Extension mechanism 122 can also be coupled between support base 90 and support member 96 or drive assembly 104.

As with braking mechanism 116, extension mechanism 122 is optionally coupled to exercise cycle controller 120. Exercise cycle controller 120 controls the operation of extension mechanism 122, and thus the tilt of upright support structure 92 in response to various user inputs or other control signals.

In one embodiment, upon contraction of extension mechanism 122, support member 94 is lowered, causing upright support structure 92 to tilt backward so that seat 98 is below the neutral position. When extension mechanism 122 is selectively extended to an extended position, support member 94 is raised, causing upright support structure 92 to tilt forward so that seat 98 is above the neutral position. Through the forward and backward tilting of upright support structure 92, as described above, exercise cycle 12b is able to more closely simulate for a user the experience of riding a bicycle on level ground as well as up and down hills.

Attention is now directed back to system 10 and how system 10 simulates an outdoor exercise experience. More specifically, the following discussion will be directed toward how system 10 enables a user to i) select a real world route, trail, path, or course, ii) exercise on an exercise device 12 that simulates the terrain of the selected real world route, trail, path, or course, and iii) view images of the real world route, trail, path, or course while exercising on the exercise device 12. While the following discussion will be directed toward using treadmill 12a with system 10, it will be appreciated from the disclosure herein that other types of exercise devices, such as exercise cycle 12b, can be used with system 10.

As shown in Figure 2, treadmill 12a includes control panel 22 attached to side members 28, 30 of upright support structure 24. Control panel 22, in one embodiment, as shown in Figure 6, includes one or more interface devices. Such interface devices may be either input devices or output devices. Input devices enable a user to input and vary the operating parameters of treadmill 12a. As examples of such input devices, control panel 22 includes many typical controllers for use on an exercise device, such as a treadmill. A number of illustrative input devices include but are not limited to time controls 126, distance controls 128, speed controls 130, incline controls 132, a start button 134, a stop or pause button 136, and heart rate controls 138. In addition to these input devices, such as one or more controllers, control panel 22 further optionally includes an iFit.com button 140, a manual override button 142, and a scaling control 144, each of which are also examples of input devices. It may be appreciated that each of the above-recited controllers or buttons may be embodied in a variety of different manners to perform their commonly utilized function. In addition, each controller, button, and the like may take the form of one or more switches, rheostats, potentiometers, touch sensitive controls, voice activated controllers, and the like. The input devices described herein are examples of structures capable of performing the function of interface means for gathering a first signal (such as a real time signal) from the user. One skilled in the art may identify various other configurations of interface means that are capable of performing the desired function.

In addition to the above-described input devices control panel 22 may include a variety of other input devices. For example, control panel 22 may include an integrally formed mouse 146. Additionally, control panel 22 may include a keyboard jack 148 for an external keyboard 150 (Figure 2), a touch-sensitive video display 152, and various other ports, jacks, or the like to receive various other external components. It will be appreciated that the external components, such as keyboard 150, may be integrally formed within control panel 22. Additionally, one or more of the input devices may be incorporated into personal computer 16 (Figure 1).

Each input device is adapted to allow a user operating treadmill 12a to more fully operate one or more operating parameters of treadmill 12a. Furthermore, the input devices enable the user to access communication system 14 and/or obtain maps, topographical information, pictures or videos of real world places, or other information via network 18, whether such information is from communication system 14, one or more third parties 20, or from one of a variety of other hardware and/or software modules that are accessible via network 18. For example, the input devices may allow the user to access the Internet to find maps, topographical data, pictures, and/or videos of real world locations, routes, paths, courses, and the like. These additional input devices are further examples of structures capable of performing the function of interface means, communicating with the exercise mechanism, for gathering a first signal from the user.

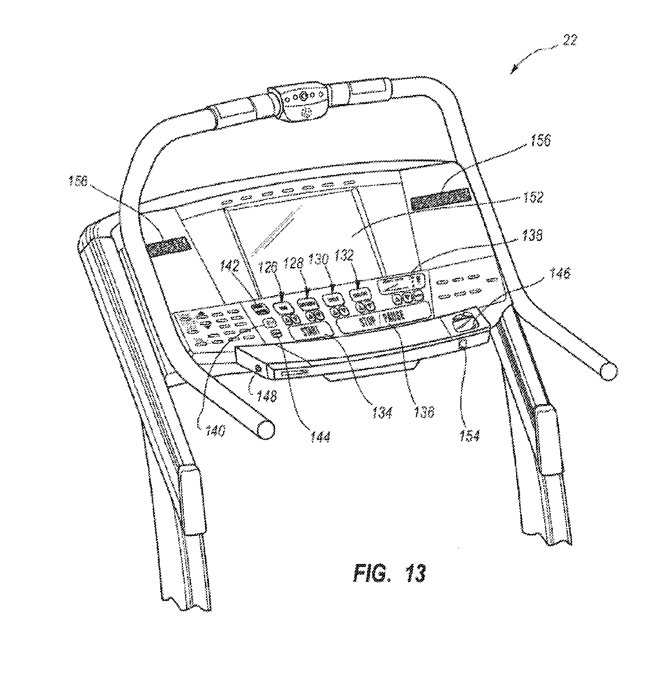

As shown in Figure 13, the iFit.com button 140, in one embodiment, acts as both a selector and an indicator of connectivity of treadmill 12a to communication system 14, and optionally one or more third parties 20, whether such connectivity is via computer 16, wireless router 17, or directly from treadmill 12a. The iFit.com button 140 optionally includes an indicator light (not shown) that demonstrates when a connection has been established between treadmill 12a and communication system 14, such as when the iFit.com button 140 is depressed. Alternatively, a light emitting diode (LED) positioned in close proximity to the iFit.com button 140 may be activated when the iFit.com button 140 is activated.

As discussed above, the connection achieved by activating iFit.com button 140 may be via a variety of communication line connections. For example, as shown, control panel 22 includes a wireless port 154 that enables treadmill 12a to wirelessly communicate with network 18 (Figure 1), either directly or via computer 16 and/or wireless router 17. Alternatively, control panel 22 may have a hard wire connection to network 18, either directly or via computer 16.

In one embodiment, by activating iFit.com button 140, a user of treadmill 12a, or other exercise device, connects to communication system 14, such as a website. Such connection may be via an independently located computer, such as computer 16, through a modem (not shown), wireless router 17, or directly through a local area network (LAN) or wide area network (WAN) by way of the described communication line connections for example, or other connections known to one skilled in the art. More specifically, by activating the iFit.com button 140 a signal is transmitted via network 18 to communication system 14 to create a connection therebetween.

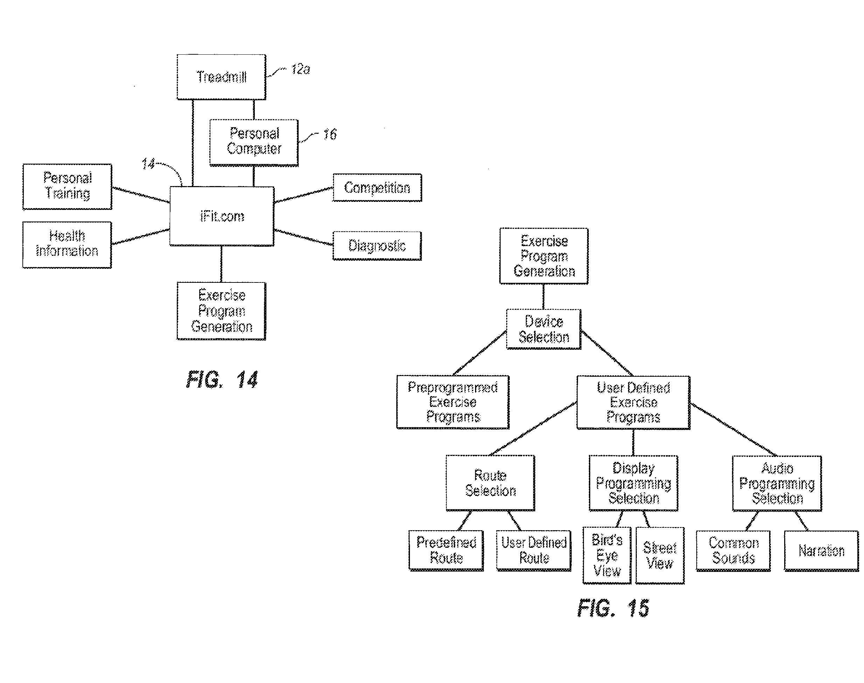

As illustrated in Figure 14, once a connection is made between treadmill 12a and communication system 14, a user may access various programs, features, and the like of communication system 14. For example, once a connection is made, a user can access, select, create, and/or download exercise programming for use with treadmill 12a. As discussed below, the exercise programming can include one or more signals that are able to adjust one or more operating parameters of treadmill 12a as well as video and/or audio programming. With an established connection to communication system 14, the user can also select other options, such as personal training, health information, competition, diagnostics, and the like, as shown in Figure 14.

In another embodiment, a user may access communication system 14 using personal computer 16. With a connection established between personal computer 16 and communication system 14, a user may access the programs, features, and options mentioned above. After the user selects the desired option, such as selecting an exercise program, communication system 14 can communicate the exercise program to treadmill 12a directly or via personal computer 16 or a portable memory device, such as a Secure Digital (SD) memory card.

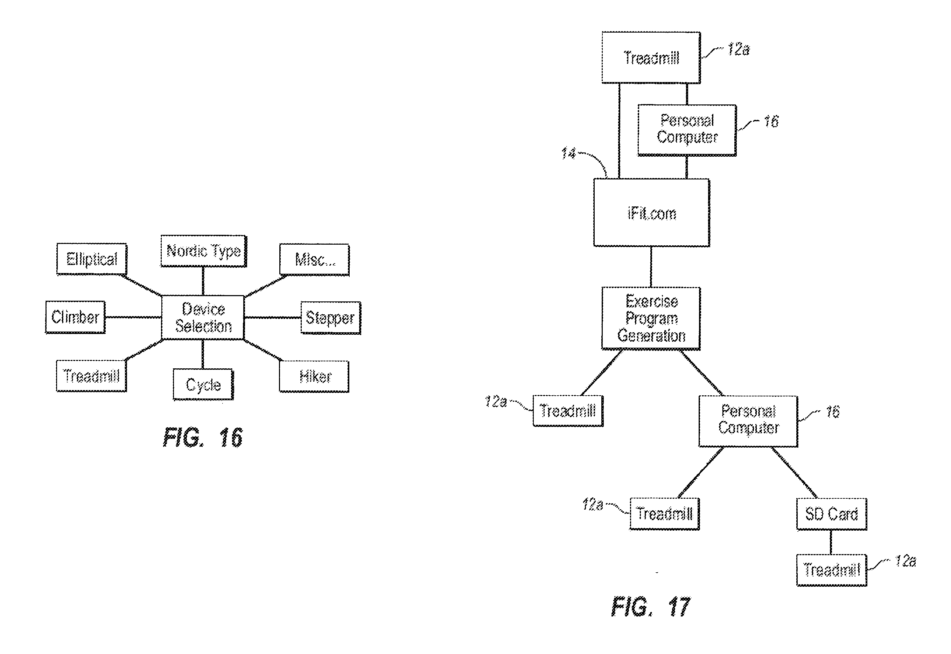

In either embodiment, and as illustrated in Figure 15, when a user indicates that he or she would like to select and download an exercise program, communication system 14 may prompt the user to select the type of exercise device upon which the exercise program will be used. Figure 16 illustrates a functional block diagram of the process of selecting an exercise device to be used in connection with the exercise program. As seen in Figure 16, the user may be given the option to select from among many different types of exercise devices, including treadmills, cycles, Nordic type skiers, climbers, hikers, steppers, ellipticals, and the like. After selecting the type of exercise device to be used, the user can then select the desired exercise program that is compatible with the selected exercise device.

The exercise programming can take any one of a number of forms. The exercise programming can include signals generated by communication system 14 and sent to treadmill 12a. The signals may include exercise control signals, audio programming, and/or display programming. The exercise controls signals can be configured to control/adjust one or more operating parameters of treadmill 12a, such as the exercise time, the incline and/or tilt of treadbase 26, and/or the speed of belt 42. The video and/or audio programming can provide various types of information, including instruction, education, and entertainment.

As illustrated in the embodiment of Figure 15, the user may select between preprogrammed exercise programming and user defined exercise programming. When a user selects the preprogrammed exercise programming option, the user can view and select from among one or more available preprogrammed exercise programs. The signals of the selected exercise programming can then be sent over network 18 to treadmill 12a to control the operating parameters of treadmill 12a and/or provide video/audio programming to the user. The exercise programming may include common exercise routines that vary the speed and incline of treadmill 12a at various time intervals during the routine.

The exercise programming may also be adapted to simulate a real world environment, such as a trail, route, course, path, or the like. By way of non-limiting example, the exercise programming may be adapted to simulate the New York City Marathon. More specifically, the control signals can be adapted to adjust the incline and tilt of treadbase 26 to replicate the hills, level surfaces, and the like, encountered on the route of the race.

In addition to adjusting the physical operating parameters of treadmill 12a, the exercise programming can include video and/or audio programming that is related to the control signals. The display programming can be presented on video display 152, while the audio programming can be presented by audio output 156, such as a speaker. In the example of the exercise programming simulating the New York City Marathon course, the display programming can include still or moving images of the course, including buildings, bridges, and roads that are seen along the course.

The display programming can be synchronized with the control signals that adjust the operating parameters of treadmill 12a. Synchronizing the control signals and the display programming allows a user to view the real world environment at the same time the user encounters operating parameters that simulate the viewed real world environment. For example, as a user runs on treadmill 12a, the control signals may cause treadmill 12a to simulate the terrain (i.e., hills, etc.) that a runner would encounter as he or she runs through Central Park. As the user of treadmill 12a experiences the terrain of Central Park, the user can also view images, whether still or moving, of the area of Central Park which the control signals are simulating.

Similar to the display programming, the audio programming can include typical sounds heard by a runner in the race, including the cheers of the crowd, cars, sirens, horns, and the like. The audio programming may also provide information about the images presented in the display programming. For example, the audio programming may include information typically provided on tour of New York City. The audio programming can be synchronized with the control signals and the display programming so that the sounds and/or information provided by the audio programming is related to what the user is seeing on video display 152 and experiencing on treadmill 12a. For instance, as the user views images and experiences the terrain of Central Park, the user may also hear sounds typical of Central Park, such as children playing, dogs parking, and people talking. Additionally, or alternatively, the user may be provided with information in narrative form about Central Park, such as its size, history, or other interesting facts.

While the exercise programming has been described above in connection with the New Your City Marathon, it will be appreciated that the exercise programming may simulate other real world environments, such as other races, mountain hikes, city tours, or any other course, route, path, and the like.

Alternatively, as illustrated in Figure 15, the user may select the user defined exercise programming option. Communication system 14 may be configured to allow a user at treadmill 12a or personal computer 16 to create exercise programming suitable to the desires of the user. When creating a user defined or unique exercise program, the user may have the option to select, among other things, a desired route, display programming, and/or audio programming. The user may also have the option to select other exercise programming parameters, such as the exercise time, changes in the speed of belt 42, changes in the incline and/or tilt of treadbase 26, and the like.

By way of example, a user accessing communication system 14 may be able to select a real world environment which he or she would like treadmill 12a to simulate. In selecting the real world environment, the user may select a starting point, and ending point, and a specific route between the two. Alternatively, the user may select a starting point and an ending point, and allow communication system 14 and/or one or more of third parties 20 to select the route therebetween. In still other embodiments, the user may select a starting point and allow communication system 14 and/or one or more of third parties 20 to select a route that proceeds from the starting point for a selected time, distance, or the like. As noted above, the real world environment may be a famous or well known race course, tour route, hike, or the like. Alternatively, a user may create an exercise program that simulates any other real world environment.

For instance, during good weather, a user of treadmill 12a may also like to run outdoors along a route the user has developed, such as along the roads in his or her neighborhood. During bad weather, however, the use may prefer to run on treadmill 12a. In such a case, the user may access communication system 14 and create an exercise program that simulates his or her neighborhood route. More specifically, the exercise program can include controls signals that adjust the operating parameters of treadmill 12a to simulate the actual terrain (i.e., hills and level surfaces) of the neighborhood route. Additionally, exercise programming can also provide display programming showing images of the neighborhood route. As with the preprogrammed exercise programs, the display programming can be synchronized with the control signals so that a user is able to view images of the real world environment associated with the actual terrain that is simulated on treadmill 12a.

In order to generate exercise programming as described above, communication system 14 may require access to one or more types of data. Some types of data that may be needed to generate the above described exercise programming include maps, topographical data, video or image data, audio data, and the like. The map data allows the user to create a route through a real world environment which will be simulated on treadmill 12a. The topographical data can be used to generate control signals that adjust one or more operating parameters of treadmill 12a to simulate the actual topography along the real world route. The video/image data and the audio data can be used to provide the user with a visual representation of and/or audio information relating to the real world route that is simulated on treadmill 12a.

The data used to generate the exercise programming may be stored at communication system 14 or at one or more other locations. For example, communication system 14 may communicate with one or more third parties 20 which store data. Third parties 20 may be websites and/or databases that are accessible via network 18. The following are a few examples of third parties 20 that can be accessed to retrieve information and data that can be used to generate the above described exercise programming.

There are multiple route planning and mapping software applications and programs which can be used by communication system 14 and/or a user at treadmill 12a to develop a route for exercise programming as described herein. Examples of such are MAPQUEST.COM, MAPS.GOOGLE.COM, and GOOGLE EARTH (available at earth.google.com). With these applications, a user is able to select a starting point and an ending point. The applications provide multiple different routes between the two points. Alternatively, the applications allow for the creation of customized routes between the beginning and end points by selecting intermediate points between the beginning and ending points.

Similarly, there are multiple databases that store topographical data for specific regions of the world. In addition, the U.S. Geological Survey maintains a database, the GTOPO 30 or Global Topography at 30 arc/second database (available at edc.usgs.gov), which includes topographical data for the entire world. Communication system 14 can access one or more of these databases to retrieve information and data regarding the real world route that is to be simulated on treadmill 12. With this data, communication system 14 can generate the control signals that control one or more of the operating parameters of treadmill 12a, such as the incline and/or tilt of treadbase 26, to simulate the terrain of the real world route.

Databases that store still or moving images of real world locations can also be accessed by communication system 14 in order to provide to the user of treadmill 12a a visual representation of the real world route that is simulated on treadmill 12a. Examples of such databases include the GOOGLE EARTH, GOOGLE STREET VIEW (available at MAPS.GOOGLE.COM), and MICROSOFT VIRTUAL EARTH (available at www.microsoft.com/virtualearth) databases. These databases provide a bird's eye view or a street level view of a selected route or location.

With access to at least some of the data described above, communication system 14 is able to generate exercise programming that allows treadmill 12a to simulate real world environments. In one embodiment, a user of treadmill 12a accesses communication system 14 via control panel 22. Communication system 14 provides a user interface that allows the user to select a preprogrammed exercise route or create a user defined exercise route. In the case of creating a user defined exercise route, communication system 14 allows the user to enter a starting point, an ending point, and/or one or more intermediate points that will defined the exercise route.

With the supplied starting point, ending point, and/or one or more intermediate points, communication system 14 communicates with one or more third parties 20 that provide map and topographical data relating to the selected route. The map and topographical data provided by the third parties 20 may include a map highlighting the selected route, total route distance, route directions, travel times for specific speeds, as well as forward, backward, and side-to-side elevation changes along the selected route.

Communication system 14 can also communicate with one or more other third parties 20 to retrieve other data relating to the selected route. Communication system 14 can, for example, communicate with the GOOGLE STREET VIEW database to retrieve images of the selected route. Furthermore, communication system 14 can access other types of databases, such as audio databases, that provide audible information relating to the selected route.

Once communication system 14 has retrieved the desired information for the selected route, communication system 14 compiles the gathered data and generates the exercise program. Communication system 14 uses the map and topographical data to generate a series of control signals that control one or more operating parameters of treadmill 12a. In other words, using a correlation algorithm, communication system 14 can synchronize the topographical data with the map data to correlate the distance and the grade or elevation change between two points on the selected route and generate a control signal that will cause treadmill 12a to simulate that terrain. For instance, communication system 14 can use the map data to determine that the distance between point A and point B is 0,8 km (½ mile), and can use the topographical data to determine that the area between points A and B has a grade of 12%. Using this information, communication system generates one or more control signals that will cause treadmill 12a to incline treadbase 26 to a 12% grade until the user has walked for 0,8 km (½ mile). In a similar manner, communication system 14 can use the map data, the topographical data, and other reference points along the selected route to generate control signals that control the tilt of treadbase 26.

In addition to generating the control signals, communication system 14 can also generate display programming to accompany the control signals. As mentioned above, the display programming can include still or moving images of the selected real world route, which communication system 14 retrieves from one or more third parties 20. For example, communication system 14 communicates with a third party 20, such as the Google Street View database via the Google Maps API (application programming interface), to retrieve a series of images from of the selected real world route. When a series of images are used to provide a visual depiction of the selected rout, the images can be cached or buffered so that upon delivery to the user of treadmill 12a, the images provide an almost seamless, video-like depiction of the selected real world route.

As mentioned above, communication system 14 can synchronize the display programming with the control signals. In this manner, the control signals will adjust the operating parameters of treadmill 12a at the same time the display programming depicts a change in the terrain of the real world route. For instance, at the same time the control signals begin to cause treadbase 26 to incline to simulate a hill on the real world route, the display programming shows one or more images of the hill on the real world route as if the user were actually beginning to ascend the hill.

Once remote communication system 14 has generated the control signals from the topographical/map data and the display programming from the retrieved images of the real world route, remote communication system 14 can employ a correlation algorithm to synchronize the control signals with the display programming. In one embodiment, the correlation algorithm uses data about the series of retrieved images, such as the number of images along the real world route, the real world distances between the images, and the like. Similarly, the correlation algorithm also uses information from the retrieved topographical/map data, such as distances between locations on the real world route, changes in elevation between locations on the real world route, directional changes along the real world route, and the like. Using this data, the correlation algorithm synchronizes the control signals and the display programming. For example, the correlation algorithm may coordinate the first control signal with the display of the first image of the remote real world route. The correlation algorithm may correlate a subsequent image with a change in the map data, such as when the map data indicates a change in a certain distance from the previous real world location. The correlation algorithm may also correlate subsequent images with a change in the topographical data, such as an elevation change from the previous real world location.

In some embodiments, communication system 14 also provides audio programming that is synchronized with the control signals and the display programming. The audio programming can include sounds that may typically be heard along the real world route, such as cars, sirens, animals, people, and the like. The audio programming can also include in narrative form information about sites along the real world route. For example, if a user chose to have treadmill 12a simulate a route through Washington D.C. which passed by sites such as the White House, the U.S. Capital building, the Lincoln Memorial, and the Washington Monument, the audio programming could provide information about each of these sites, such as might be heard during a tour of Washington D.C. A correlation algorithm, such as the one described above, may be used to coordinate the presentation of the audio programming with the display programming and the control signals.

As shown in the flow diagram of Figure 17, in one exemplary embodiment, a user may access remote communication system 14 directly from treadmill 12a (using control panel 22), from personal computer 16, or from treadmill 12a via personal computer 16. Connection between remote communication system 14 and treadmill 12a and/or personal computer 16 can be achieve via network 18, as described herein. Network 18 and remote communication system 14 in this embodiment are the Internet and a website, respectively. Once a connection has been established with remote communication system 14 and the user has indicated that he/she would like to create exercise programming, the user may define a remote, real world exercise route by entering a starting point, an ending point, and/or one or more intermediate points. With the remote, real world exercise route defined by the starting point, ending point, and/or one or more intermediate points, remote communication system 14 accesses one or more third parties 20 to retrieve data relating to one or more characteristics of the defined remote, real world exercise route. Remote communication system 14 then uses the retrieved data to generate exercise programming for treadmill 12a.

In one embodiment, remote communication system 14 may access MAPQUEST.COM to obtain map data, including distances, directions, and the like, relating to the defined remote, real world exercise route. Remote communication system 14 may also access a database, such as the GTOPO 30 database, that stores topographical data relating to the defined remote, real world exercise route. Remote communication system 14 can use the map data and topographical data retrieved from these third parties 20 to generate control signals that will cause treadmill 12a to simulate the terrain of the remote, real world exercise route. In addition, remote communication system 14 may access a database, such as the GOOGLE STREET VIEW database, to retrieve a plurality of sequential static images of the remote, real world exercise route.

With the control signals generated from the topographical data and the images of the remote, real world exercise route, remote communication system 14 generates exercise programming for treadmill 12a as described above. Remote communication system 14 then communicates the exercise programming to treadmill 12a as shown in Figure 17. In some embodiments, remote communication system 14 can communicate the exercise programming directly to treadmill 12a, as shown in Figure 17. For example, when treadmill 12 is adapted to communicate directly with network 18, remote communication system 14 can send the exercise programming 14 directly to treadmill 12a via network 18.

In other embodiments, remote communication system 14 communicates the exercise programming to treadmill 12a via personal computer 16. For instance, when personal computer 16 is adapted to communicate with network 18 and treadmill 12a, via a hardwire or wireless connection, remote communication system 14 can send the exercise programming to personal computer 16, which can in turn send the exercise programming to treadmill 12a. Personal computer 16 can send the exercise programming to treadmill 12a through a variety of means. For example, personal computer 16 can communicate with treadmill 12a via a hardwire or wireless connection as described herein. Alternatively, personal computer 16 may be adapted to store the exercise programming on a portable memory device, which can be selectively associated with treadmill 12a. By way of non-limiting example, personal computer 16 can be adapted to receive and store the exercise programming on a portable memory device, such as an SD card, a DataFlash card, a MultiMedia Card (MMC), CompactFlash card, a removable NAND-type flash memory (e.g. SmartMedia, Sony Memory Stick), a one-time-programmable memory cards (OTP), XD cards, and the like. The portable memory device can then be removed from personal computer 16 and inserted or otherwise associated with treadmill 12a.

Once the exercise programming has been delivered to treadmill 12a via any suitable means, such as those described herein, treadmill 12a can run/execute the exercise programming by processing the control signals, the display programming, and/or the audio programming. As treadmill 12a runs the exercise programming, treadmill 12a simulates the remote, real world exercise route. In particular, the control signals of the exercise programming cause treadmill 12a to adjust one or more operating parameters, such as the incline or tilt of treadbase 26, to replicate the terrain of the remote, real world exercise route. In addition, treadmill 12 a displays, via display 152, the plurality of sequential static images of the remote, real world exercise route. As noted herein, the generation of the exercise programming includes the synchronization of the control signals and the plurality of sequential static images. This synchronization allows the user to view the images of the remote, real world exercise route while treadmill 12a simulates the terrain of the remote, real world exercise route that is associated with those images. In other words, synchronizing the control signals and the plurality of sequential static images allows a user of treadmill 12a to experience the terrain of the remote, real world exercise route while simultaneously viewing images of the portion of the remote, real world exercise route that is being simulated at that time.

Thus, in one embodiment, i) topographical data retrieved from a third party 20, such as the GTOPO 30 databases, is used to generate control signals that adjust operational parameters of treadmill 12a to simulate real world terrain; and ii) image data retrieved from another third party 20, such as the GOOGLE STREET VIEW database, is synchronized with the control signals and displayed on video output device 152. In this embodiment, the user can experience the topographical changes of the real world terrain as represented by the topographical data retrieved from the GTOPO 30 database, for example, while simultaneously viewing the corresponding images for the same terrain that have been retrieved from the GOOGLE STREET VIEW database, for example.

As noted above, treadmill 12a can monitor the actual operating parameters of treadmill 12a, such as the incline and tilt of treadbase 26 and the speed of belt 42. The actual operating parameters of treadmill 12a and the exercise programming can be correlated so that the control signals, display programming, and audio programming are updated or changed at the appropriate times. For instance, the exercise programming may include control signals that incline treadbase 26 to a 5% grade for 402 m (¼ mile) and then decline treadbase to a 2% grade for 1609 m (1 mile). The speed of belt 42 will affect the amount of time that each of the controls signals is active. If belt 42 were moving at 0,8 km per hour (Kmh) (5 miles per hour (mph)), for example, the user would traverse the 402 m (¼ mile) segment in 3 minute and the 1,6 km (1 mile) segment in 12 minutes. If belt 42 were moving at 1,17 m/s (2.5 mph), however, it would take the user 6 minutes to traverse the 402 m (¼ mile) segment and 24 minutes to traverse the 1,6 km (1 mile) segment. Thus, correlating or synchronizing the actual operating parameters of treadmill 12a with the exercise programming allows treadmill 12a to be controlled in such a way as to realistically simulate the real world environment. The correlation or synchronization of the exercise programming and the actual operating parameters can be performed by treadmill controller 50, control panel 22, personal computer 16, communication system 14, or a combination thereof.

As mentioned above, control panel 22 may include manual override button 142. Manual override button 142 enables a user to override a control signal generated by communication system 18, personal computer 16, or treadmill 12a. For example, if the exercise program accessed through communication system 14 is too difficult for the user, the user may activate manual override button 142, thereby interrupting or decreasing the difficultly level of the program delivered to treadmill 12a by communication system 14. Furthermore, in the event that the exercise program is too easy, the user may increase the difficulty level of the exercise device. Consequently, manual override button 142 provides the user with a safety switch during operation of treadmill 12a. In an alternate configuration of treadmill 12a, the functionality of manual override button 142 is activated upon manual activation of one of the other input devices, such as but not limited to, time controls 126, distance controls 128, speed controls 130, incline controls 132, stop/pause button 136, and the like.

Similar to the operation of manual override button 142, scaling control 144 enables a user to vary the operating parameters of treadmill 12a during an exercise program run by treadmill 12a. A user may activate scaling control 144 and vary the intensity of an exercise program. The scaling control 144, therefore, enables a user to select a value representative of the proportional change to be made to the control signal received by the communicating mechanism of treadmill 12a from communication system 14. For example, if an exercise program requires a maximum speed of 8,6 kmh (6 mph) with a maximum incline of 15 degrees for a period of 30 minutes, an individual may activate scaling control 144 to require only 66% intensity of the exercise program; stated otherwise, reduce the intensity by one third. Therefore, the exercise program is varied to a maximum speed of 6,4 kmh (4 mph), with a maximum incline of 10 degrees, for a period of 20 minutes. Optionally, scaling control 144 may enable the user to set maximum values for each operating parameter of treadmill 12a. In another configuration, scaling control 144 may enable the user to scale only one operating parameter of treadmill 12a while leaving other parameters unchanged. Hence, the user may vary the exercise program to their particular abilities, while obtaining the beneficial effects of exercising.

In addition to the output devices described above, the present invention may include various other output devices to provide information and data to the user of treadmill 12a. In one embodiment of treadmill 12a, control panel 22 includes one or more operating parameter displays. The one or more operating parameter displays give a visual display of some of the more important exercise device operating parameters, such as, but not limited to, speed, incline, distance traveled, calories used, elevation climbed, wheel resistance, and the like. The one or more operating parameter displays may use a numerical display, a graphical display, combinations thereof, or such other displays known to one skilled in that art. For example, the operating parameter display may be incorporated within video output device 152.

According to yet another aspect of the present invention, the exercise device is capable of being controlled by signals from the communication system and/or physical controls integrated onto the exercise device. The physically integrated controls and the controls from the communication system may be passed through a buffer that controls the exercise device. In this way, in the event that connectivity to the communication system is lost, a user of the exercise device would still be capable of controlling the exercise device.

As used in this specification and the appended claims, the phrases "communicating with," and "in communication with" and similar phrases shall mean any type of applicable communication known to one skilled in the art in light of the disclosure herein, such as electrical communication, optical communication, physical communication, magnetic communication, software communication, hardware communication, data communication, and the like.

An exercise system according to one embodiment of the present invention is configured to simulate a real world exercise route. The exercise system of the present embodiment comprises an exercise device including a movable element for movement in performance of exercise by a user and at least one actuator for controlling one or more operating parameters of the exercise device. The exercise device is adapted to receive exercising programming and use the exercise programming to substantially simulate one or more aspects of the real world exercise route. The exercise programming includes one or more control signals representative of changes to be made to the one or more operating parameters to substantially simulate the real world exercise route. The exercise system also includes a remote communication system adapted to use data relating to the real world exercise route to generate the exercise programming. The data is stored external to the remote communication system. The system also includes a network adapted to facilitate communication of the exercise programming from the remote communication system to the exercise device.

According to the present embodiment, the data used to generate the exercise programming comprises map data, topographical data, video or image data, or a combination thereof. The communication system is adapted to communicate with at least one third party to obtain the data used to generate the exercise programming. The at least one third party comprises a database or website. The exercise device of the exercise system is adapted to communicate with the network either directly or through a separate computer. The exercise device is selected from the group consisting of a treadmill, a exercise cycle, a climber, a hiker, an elliptical, and a stepper.

The exercise programming includes display programming including images of the real world exercise route. Furthermore, the control signals are adapted to control the one or more operating parameters to substantially simulate topographical characteristics of the real world exercise route. The control signals control the one or more operating parameters while the user views the images provided by the display programming on an exercise device display.

In another embodiment of the present invention, an exercise system is configured to simulate a real world exercise route and comprises an exercise device comprising a movable element for movement in performance of exercise by a user. The exercise device has one or more operating parameters that are controlled by exercise programming. The exercise system also includes a remote communication system adapted to communicate with a user of the exercise device to enable the user to select a starting point and an ending point for the real world exercise route of user defined exercise programming and generate the user defined exercise programming that includes one or more control signals representative of changes to be made to the one or more operating parameters to substantially simulate topographical characteristics of the real world exercise route, and display programming including images of the real world exercise route. The remote communication system generates the exercise programming from data stored external to the remote communication system. Additionally, the exercise system includes a network in communication with the exercise device and the remote communication system, the network being configured to communicate the exercise programming from the remote communication system to the exercise device.

In the present embodiment, the exercise device comprises a treadmill having a base frame, a treadbase mounted on the base frame, and an endless belt trained around the treadbase to enable the user to ambulate thereon. The treadbase is pivotally mounted on the base frame so that the treadbase can be selectively inclined or declined to simulate for the user the experience of ambulating up and down hills of the real world exercise route. Additionally, the treadbase is pivotally mounted on the base frame so that the treadbase can be selectively tilted from one side to the other side to substantially simulate for the user the experience of ambulating on an uneven surface of the real world exercise route.

According to the present embodiment, the one or more control signals control the operating parameters of the exercise device while the display programming is presented to the user on an exercise device display. Also, the data stored external to the remote communication system comprises data stored by at least one third party.

In a further embodiment of the present invention, an exercise system configured to simulate a remote, real world exercise route comprises an exercise device having a movable element for movement in performance of exercise by a user. The exercise device is receptive to control signals that adjust one or more operating parameters associated with the movable element to substantially simulate topographical characteristics of the remote, real world exercise route, while simultaneously presenting images of the remote, real world exercise route on an exercise device display. The exercise system also includes a remote communication system that generates exercise programming and communicates the exercise programming to the exercise device. The remote communication system uses data relating to the appearance and topographical characteristics of the remote, real world exercise route to generate the exercise programming. The exercise programming includes i) one or more control signals that are adapted to cause a change in the one or more operating parameters to substantially simulate the topographical characteristics of the remote, real world exercise route, and ii) display programming including the images of the remote, real world exercise route presented on the exercise device display. Moreover, the exercise system includes at least one database external to the remote communication system, the at least one database storing the data used by the remote communication system to generate the exercise programming.

In the present embodiment, the exercise device comprises an exercise cycle that includes: a support base adapted to rest upon a support surface; an upright support structure mounted to the support base, the upright support structure having a seat, a handle bar assembly, and a control panel; a pedal assembly adapted to be engaged and rotated by the user's feet, the pedal assembly being coupled to the upright support structure; and a resistance assembly adapted to provide resistance to the rotation of the pedal assembly, the resistance assembly being coupled to the upright support structure, the resistance assembly being controlled by the one or more control signals of the exercise programming.

The upright support structure is pivotally mounted to the support base, the upright support structure being selectively tilted forward or backward to substantially simulate for the user the experience of riding a bicycle up or down a hill. The resistance provided by the resistance assembly can be associated with the tilt of the upright support structure to substantially simulate for the user the experience of riding a bicycle up or down a hill on the remote, real world exercise route. The exercise programming is adapted to adjust the tilt of upright support structure and the resistance provided by the resistance assembly based upon the topographical characteristics of the remote, real world exercise route and the user's weight.

Yet a further embodiment of the present invention relates to an exercise system configured to simulate a remote, real world exercise route. The exercise system includes a network and an exercise device in communication with the network. The exercise device is receptive to exercise programming that is adapted to control one or more selectively adjustable operating parameters of the exercise device to substantially simulate the remote, real world exercise route. The system also includes at least one external database in communication with the network, the at least one database storing data relating to one or more characteristics of the remote, real world exercise route, and a remote communication system adapted to generate the exercise programming and communicate the exercise programming to the exercise device. The remote communication system is adapted to allow a user of the exercise device to define the remote, real world exercise route. The remote communication system is also adapted to communicate with the at least one database via the network to retrieve the data relating to the one or more characteristics of the remote, real world exercise route. Further, the remote communication system is adapted to use the user defined remote, real world exercise route and the data retrieved from the at least one database to generate the exercise programming, wherein the at least one database is external to the remote communication system.

In the present embodiment, the data stored by the at least one database comprises map data, topographical data, image data, or a combination thereof. The remote communication system is adapted to generate one or more control signals for incorporation in the exercise programming, the one or more control signals being generated based on the topographical data retrieved from the at least one database. The one or more control signals are representative of changes to be made to the one or more selectively adjustable operating parameters of the exercise device to substantially simulate the remote, real world exercise route. Furthermore, the remote communication system is adapted to generate display programming for incorporation in the exercise programming, the display programming including the image data retrieved from the at least one database. Additionally, the at least one database is external to and remote from the remote communication system.

In another embodiment of an exercise system configured to simulate a remote, real world exercise route, the exercise system comprises an exercise device and a remote communication system. The exercise device is adapted to substantially simulate the remote, real world exercise route in response to exercise programming, wherein the exercise device comprises a user interface that enables a user to define the remote, real world exercise route by selecting one or more parameters of the remote, real world exercise route. The one or more user selectable parameters of the remote, real world exercise route include a starting point, an ending point, a total distance, a total elevation change, or a combination thereof The remote communication system in is communication with the exercise device. The remote communication system is adapted to receive the one or more user defined parameters of the remote, real world exercise route and communicate with at least two third parties to retrieve data stored by the at least two third parties relating to the remote, real world exercise route. The remote communication system is also adapted to use the data retrieved from the at least two third parties to generate the exercise programming that includes control signals for controlling the operation of the exercise device and display programming synchronized with the control signals.

The remote communication system communicates with the at least two third parties via a network. The at least two third parties comprise at least two databases external to and remote from the remote communication system. The at least two databases are accessible via one or more websites. The remote communication system uses topographical data stored by one of the at least two third parties to generate the control signals that are configured to adjust one or more operating parameters of the exercise device. Similarly, the remote communication system uses image data stored by another one of the at least two third parties to generate the display programming. The image data comprises a plurality of sequential static images of the remote, real world exercise route.

The synchronization of the display programming and the control signals provides the user with the ability to view images of the real world exercise route while substantially experiencing, by way of the one or more changing operating parameters, the topographical characteristics of the real world environment viewed in the images of the display programming. The remote communication system synchronizes the control signals and the display programming so that there is a temporal relationship between changes in the control signals and the display programming.

A first third party of the at least two third parties of the present embodiment stores topographical data relating to the remote, real world exercise route, a second third party of the at least two third parties stores a plurality of sequential static images of the remote, real world exercise route, and the remote communication system synchronizes the plurality of sequential static images with control signals representative of the topographical data. Alternatively, a first third party of the at least two third parties stores topographical data relating to the remote, real world exercise route, a second third party of the at least two third parties stores one or more video images of the remote, real world exercise route, and the remote communication system synchronizes the one or more video images with control signals representative of the topographical data.

Another embodiment of the present invention relates to a method for generating exercise programming for substantially simulating a remote, real world exercise route on an exercise device. The method can be practiced in an exercise system having a remote communication system adapted to communicate with the exercise device and at least two third party databases. The method includes the steps of: receiving one or more parameters for defining the remote, real world exercise route; retrieving a first type of data relating to the remote, real world exercise route from a first third party database of the at least two third party databases; retrieving a second type of data relating to the remote, real world exercise route from a second third party database the at least two third party databases; generating one or more control signals using the first type of data retrieved from the first third party database, the control signals being adapted to cause the exercise device to substantially simulate topographical characteristics of the remote, real world exercise route; generating display programming using the second type of data retrieved from the second third party database, the display programming including images of the remote, real world exercise route; and synchronizing the one or more control signals and the display programming.

The method can also include communicating the one or more remote, real world exercise route defining parameters to the at least two third party databases, or communicating the one or more remote, real world exercise route defining parameters from the exercise device to the remote communication system. The method can also include generating a remote, real world exercise route based on the one or more remote, real world exercise route defining parameters. The step of synchronizing the one or more control signals and the display programming comprises temporally relating the one or more control signals and the display programming. The step of synchronizing the one or more control signals and the display programming correlates changes in the one or more control signals to changes in the display programming.

Each of the first and second third party databases comprises one or more databases accessible by the remote communication system via a website. The first type of data comprises topographical data, and the second type of data comprises image data and the second type of data is used to generate the display programming includes image data. The image data comprises one or more static images and/or one or more video images.

In still yet another embodiment, an exercise system configured to substantially simulate a remote, real world exercise route comprises a network, an exercise device, a plurality of third parties, and a remote communication system. The exercise device is in communication with the network, the exercise device being receptive to exercise programming that is adapted to control one or more selectively adjustable operating parameters of the exercise device to substantially simulate the remote, real world exercise route. The plurality of third parties is in communication with the network, the plurality of third parties storing data relating to one or more characteristics of the remote, real world exercise route. The plurality of third parties comprises one or more databases that store topographical data relating to the remote, real world exercise route, and one or more other databases that store image data relating to the remote, real world exercise route. The remote communication system is external to and remote from the plurality of third parties, the remote communication system is adapted to allow a user of the exercise device to define the remote, real world exercise route. The remote communication system is adapted to communicate with the plurality of third parties via the network to retrieve the data relating to the one or more characteristics of the remote, real world exercise route. The remote communication system is also adapted to use the data retrieved from the plurality of databases to generate the exercise programming. Generating the exercise programming includes (i) using the image data to generate display programming; (ii) using the topographical data to generate control signals; and (iii) synchronizing the display programming with the control signals, the remote communication system being adapted to communicate the exercise programming to the exercise device.

The remote communication system generates the control signals using the topographical data. The control signals control the one or more selectively adjustable operating parameters of the exercise device to substantially simulate the terrain of the remote, real world exercise route. The image data comprises a plurality of sequential static images of the remote, real world exercise route and the exercise device comprises a display adapted to display the plurality of sequential static images.

Synchronization of the control signals and the plurality of sequential static images enables the user of the exercise device to substantially experience the terrain of the remote, real world exercise route on the exercise device while simultaneously viewing images of the remote, real world exercise route associated with the simulated terrain. The image data comprises video images of the remote, real world exercise route and the exercise device comprises a display adapted to display the video images of the remote, real world exercise route. The plurality of third parties are external to and remote from the remote communication system.

The present invention may be embodied in other specific forms without departing from its spirit or essential characteristics. The described embodiments are to be considered in all respects only as illustrative and not restrictive. The scope of the invention is, therefore, indicated by the appended claims rather than by the foregoing description. All changes which come within the meaning and range of equivalency of the claims are to be embraced within their scope.

## Claims

1. An exercise system configured to simulate a real world exercise route, the exercise system comprising:
an exercise device (12) comprising a movable element for movement in performance of exercise by a user, at least one actuator for controlling one or more operating parameters of the exercise device, and a display, the exercise device being adapted to receive exercising programming and use the exercise programming to substantially simulate one or more aspects of the real world exercise route, the exercise programming including one or more control signals representative of changes to be made to the one or more operating parameters to substantially simulate the real world exercise route;
a remote communication system (14) adapted to use data relating to the real world exercise route to generate the exercise programming,
wherein the remote communication system is configured to retrieve the data, ,the data being stored external to the remote communication system,
wherein the remote communication system is adapted to generate display programming for incorporation in the exercise programming, the display programming including image data retrieved from at least one database external to the communication system, wherein the display programming is displayed on the exercise device display,
wherein the remote communication system is configured to synchronize the data and the control signals to create the exercise programming to substantially simulate the real world exercise route;
and
a network (18) adapted to facilitate communication of the exercise programming from the remote communication system to the exercise device.

2. An exercise system as recited in claim 1, wherein the image data comprises a plurality of sequential static images of the real world exercise route, and/or wherein the image data comprises video images of the real world exercise route.

3. An exercise system as recited in claim 1, wherein the control signals control the one or more operating parameters while the user views images of the real world exercise route provided by the display programming on the exercise device display.

4. An exercise system as recited in claim 1, wherein the remote communication system synchronizes the control signals and the display programming so that there is a temporal relationship between changes in the control signals and images of the real world exercise route provided by the display programming, wherein the synchronization of the display programming and the control signals provides the user with the ability to view the images of the real world exercise route while substantially experiencing, by way of the one or more changing operating parameters, the topographical characteristics of the real world environment viewed in the images of the display programming.

5. An exercise system as recited in claim 1, wherein the data used to generate the exercise programming comprises map data, topographical data, video data, image data, or a combination thereof.

6. An exercise system as recited in claim 1, wherein the remote communication system is adapted to communicate with at least two third parties to obtain the data used to generate the exercise programming.

7. An exercise system as recited in claim 6, wherein a first third party of the at least two third parties stores topographical data relating to the real world exercise route, a second third party of the at least two third parties stores video or static images of the real world exercise route, and the remote communication system synchronizes the video or static images with control signals representative of the topographical data.

8. An exercise system as recited in claim 1, wherein the remote communication system is adapted to communicate with a user of the exercise device to enable the user to select one or more user defined parameters of the real world exercise route, wherein the one or more user selectable parameters of the real world exercise route include a starting point, an ending point, a total distance, a total elevation change, or a combination thereof.

9. An exercise system as recited in claim 1, wherein the exercise device comprises a user interface that enables a user to define the real world exercise route by selecting one or more parameters of the real world exercise route; and
wherein the remote communication system is adapted for communication with the exercise device, wherein the remote communication system is adapted to receive the one or more user defined parameters of the real world exercise route and communicate with at least two third parties to retrieve data stored by the at least two third parties relating to the real world exercise route, and wherein the remote communication system is adapted to use the data retrieved from the at
least two third parties to generate the exercise programming that includes control signals for controlling the operation of the exercise device and display programming synchronized with the control signals.

10. An exercise system as recited in claim 9, wherein the remote communication system communicates with the at least two third parties via a network, the at least two third parties comprising at least two databases external to and remote from the remote communication system, wherein the at least two databases are accessible via one or more websites

11. An exercise system as recited in claim 9, wherein the remote communication system uses topographical data stored by one of the at least two third parties to generate the control signals that are configured to adjust one or more operating parameters of the exercise device, wherein the remote communication system uses image data stored by another one of the at least two third parties to generate the display programming, wherein the image data comprises a plurality of sequential static images of the real world exercise route, wherein the synchronization of the display programming and the control signals provides the user with the ability to view images of the real world exercise route while substantially experiencing, by way of the one or more changing operating parameters, the topographical characteristics of the real world environment viewed in the images of the display programming.

12. An exercise system as recited in claim 9, wherein a first third party of the at least two third parties stores topographical data relating to the real world exercise route, a second third party of the at least two third parties stores video or static images of the real world exercise route, and the remote communication system synchronizes the video or static images of the display programming with control signals representative of the
topographical data so that there is a temporal relationship between changes in the control signals and the display programming.

13. An exercise system as recited in claim 1, further comprising a plurality of third parties in communication with the network, the plurality of third parties storing data relating to one or more characteristics of the real world exercise route, the plurality of third parties comprising (i) one or more databases that store topographical data relating to the real world exercise route, and (ii) one or more other databases that store image data relating to the real world exercise route,
wherein the exercise device is adapted for communication with the network, the exercise device being receptive to the exercise programming that is adapted to control the one or more operating parameters of the exercise device to substantially simulate the real world exercise route; and
wherein the remote communication system is external to and remote from the plurality of third parties, the remote communication system being adapted to allow a user of the exercise device to define the real world exercise route, the remote communication system being adapted to communicate with the plurality of third parties via the network to retrieve the data relating to the one or more characteristics of the real world exercise route, the remote communication system being adapted to use the data retrieved from the plurality of databases to generate the exercise programming, wherein generating the exercise programming includes (i) using the image data to generate display programming, the image data comprising a plurality of sequential static images of the real world exercise route that can be displayed on the exercise device display; (ii) using the topographical data to generate control signals, the control signals being adapted to control the one or more operating parameters of the exercise device to substantially simulate the terrain of the real world exercise route; and (iii) synchronizing the plurality of sequential static
images of the display programming with the control signals, the synchronization of the control signals and the plurality of sequential static images enabling the user of the exercise device to substantially experience the terrain of the real world exercise route on the exercise device while simultaneously viewing images of the real world exercise route associated with the simulated terrain, and wherein the remote communication system is adapted to communicate the exercise programming to the exercise device.

14. A method for generating exercise programming for substantially simulating a remote, real world exercise route on an exercise device, comprising:
receiving, by a remote communication system (14), one or more parameters for defining the remote, real world exercise route;
retrieving, by the remote communication system, a first type of data relating to the remote, real world exercise route from a first third party external database of the at least two third party external databases;
retrieving, by the remote communication system, a second type of data relating to the remote, real world exercise route from a second third party external database of the at least two third party external databases;
generating, by the remote communication system, one or more control signals using the first type of data retrieved from the first third party external database, the control signals being adapted to cause the exercise device to substantially simulate topographical characteristics of the remote, real world exercise route;
generating, by the remote communication system, display programming using the second type of data retrieved from the second third party external database, the display programming including images of the remote, real world exercise route; and
synchronizing, by the remote communication system, the one or more control signals and the display programming.

15. The method as recited in claim 14, wherein the first type of data comprises topographical data and the second type of data comprises image data, and wherein synchronizing the one or more control signals and the display programming comprises temporally correlating changes in the one or more control signals with changes in the display programming.

## Patentansprüche

1. Trainingssystem, das konfiguriert ist, um eine Realwelt-Trainingsroute zu simulieren, wobei das Trainingssystem umfasst:
eine Trainingsvorrichtung (12), die ein bewegliches Element für eine Bewegung bei einer Durchführung eines Trainings durch einen Benutzer, mindestens eine Betätigungsvorrichtung zum Steuern eines oder mehrerer Betriebsparameter der Trainingsvorrichtung und eine Anzeige umfasst, wobei die Trainingsvorrichtung eingerichtet ist, um eine Trainingsprogrammierung zu empfangen und die Trainingsprogrammierung zu verwenden, um einen oder mehrere Aspekte der Realwelt-Trainingsroute im Wesentlichen zu simulieren, wobei die Trainingsprogrammierung ein oder mehrere Steuersignale umfasst, die Änderungen repräsentieren, welche an dem einen oder den mehreren Betriebsparametern vorzunehmen sind, um die Realwelt-Trainingsroute im Wesentlichen zu simulieren,
ein fernes Kommunikationssystem (14), das eingerichtet ist, um Daten, welche die Realwelt-Trainingsroute betreffen, zu verwenden, um die Trainingsprogrammierung zu erzeugen,
wobei das ferne Kommunikationssystem eingerichtet ist, um die Daten zu empfangen, wobei die Daten außerhalb des fernen Kommunikationssystems gespeichert sind,
wobei das ferne Kommunikationssystem eingerichtet ist, um eine Anzeigeprogrammierung zum Aufnehmen in die Trainingsprogrammierung zu erzeugen, wobei die Anzeigeprogrammierung Bilddaten umfasst, die von mindestens einer Datenbasis außerhalb des Kommunikationssystems abgerufen werden, wobei die Anzeigeprogrammierung auf der Trainingsvorrichtungsanzeige angezeigt wird,
wobei das ferne Kommunikationssystem eingerichtet ist, die Daten und die Steuersignale zu synchronisieren, um die Trainingsprogrammierung zu erzeugen, um die Realwelt-Trainingsroute im Wesentlichen zu simulieren; und
ein Netz (18), das eingerichtet ist, um eine Übermittlung der Trainingsprogrammierung von dem fernen Kommunikationssystem zu der Trainingsvorrichtung zu unterstützen.

2. Trainingssystem nach Anspruch 1, wobei die Bilddaten mehrere sequentielle statische Bilder der Realwelt-Trainingsroute umfassen, und/oder wobei die Bilddaten Videobilder der Realwelt-Trainingsroute umfassen.

3. Trainingssystem nach Anspruch 1, wobei die Steuersignale den einen oder die mehreren Betriebsparameter steuern, während der Benutzer Bilder der Realwelt-Trainingsroute betrachtet, die von der Anzeigeprogrammierung auf der Trainingsvorrichtungsanzeige bereitgestellt werden.

4. Trainingssystem nach Anspruch 1, wobei das ferne Kommunikationssystem die Steuersignale und die Anzeigeprogrammierung derart synchronisiert, dass eine zeitliche Beziehung zwischen Änderungen der Steuersignale und Bildern der Realwelt-Trainingsroute, welche von der Anzeigeprogrammierung bereitgestellt werden, vorhanden ist, wobei die Synchronisation der Anzeigeprogrammierung und der Steuersignale es dem Benutzer ermöglicht, die Bilder der Realwelt-Trainingsroute zu betrachten, während er im Wesentlichen mittels des einen oder der mehreren sich ändernden Betriebsparameter die topografischen Merkmale der Realwelt-Umgebung, welche auf Bildern der Anzeigeprogrammierung betrachtet werden, wahrnimmt.

5. Trainingssystem nach Anspruch 1, wobei die Daten, welche verwendet werden, um die Trainingsprogrammierung zu erzeugen, Kartendaten, topografische Daten, Videodaten, Bilddaten oder eine Kombination daraus umfassen.

6. Trainingssystem nach Anspruch 1, wobei das ferne Kommunikationssystem eingerichtet ist, um mit mindestens zwei Dritten zu kommunizieren, um die Daten zu erhalten, welche verwendet werden, um die Trainingsprogrammierung zu erzeugen.

7. Trainingssystem nach Anspruch 6, wobei ein erster Dritter der mindestens zwei Dritten topografische Daten speichert, welche die Realwelt-Trainingsroute betreffen, wobei ein zweiter Dritter der mindestens zwei Dritten Video- oder statische Bilder der Realwelt-Trainingsroute speichert, und wobei das ferne Kommunikationssystem die Video- oder statischen Bilder mit Steuersignalen synchronisiert, welche die topografischen Daten repräsentieren.

8. Trainingssystem nach Anspruch 1, wobei das ferne Kommunikationssystem eingerichtet ist, um mit einem Benutzer der Trainingsvorrichtung zu kommunizieren, um es dem Benutzer zu ermöglichen, einen oder mehrere benutzerdefinierte Parameter der Realwelt-Trainingsroute auszuwählen, wobei der eine oder die mehreren benutzerauswählbaren Parameter der Realwelt-Trainingsroute einen Startpunkt, einen Endpunkt, eine Gesamtentfernung, eine Gesamthöhenänderung oder ein Kombination daraus umfassen.

9. Trainingssystem nach Anspruch 1, wobei die Trainingsvorrichtung eine Benutzerschnittstelle umfasst, welche es dem Benutzer ermöglicht, die Realwelt-Trainingsroute durch Auswählen eines oder mehrerer Parameter der Realwelt-Trainingsroute zu definieren; und
wobei das ferne Kommunikationssystem für eine Kommunikation mit der Trainingsvorrichtung eingerichtet ist, wobei das ferne Kommunikationssystem eingerichtet ist, um den einen oder die mehreren benutzerdefinierten Parameter der Realwelt-Trainingsroute zu empfangen und mit mindestens zwei Dritten zu kommunizieren, um von den mindestens zwei Dritten gespeicherte Daten abzurufen, welche die Realwelt-Trainingsroute betreffen, und wobei das ferne Kommunikationssystem eingerichtet ist, um die von den mindestens zwei Dritten abgerufenen Daten zu verwenden, um die Trainingsprogrammierung, welche Steuersignale zum Steuern des Betriebs der Trainingsvorrichtung und die Anzeigeprogrammierung, welche mit den Steuersignalen synchronisiert ist, umfasst, zu erzeugen.

10. Trainingssystem nach Anspruch 9, wobei das ferne Kommunikationssystem mit den mindestens zwei Dritten über ein Netz kommuniziert, wobei die mindestens zwei Dritten mindestens zwei Datenbasen außerhalb des fernen Kommunikationssystems und fern von dem fernen Kommunikationssystem umfassen, wobei auf die mindestens zwei Datenbasen über eine oder mehrere Websites zugegriffen werden kann.

11. Trainingssystem nach Anspruch 9, wobei das ferne Kommunikationssystem topografische Daten, welche von einem der mindestens zwei Dritten gespeichert werden, verwendet, um die Steuersignale zu erzeugen, welche konfiguriert sind, um einen oder mehrere Betriebsparameter der Trainingsvorrichtung einzustellen, wobei das ferne Kommunikationssystem Bilddaten verwendet, welche von einem anderen der mindestens zwei Dritten gespeichert werden, um die Anzeigeprogrammierung zu erzeugen, wobei die Bilddaten mehrere sequentielle statische Bilder der Realwelt-Trainingsroute umfassen, wobei die Synchronisation der Anzeigeprogrammierung und der Steuersignale es dem Benutzer ermöglicht, Bilder der Realwelt-Trainingsroute zu betrachten, während er im Wesentlichen mittels des einen oder der mehreren sich ändernden Betriebsparameter die topografischen Merkmale der in den Bildern der Anzeigeprogrammierung betrachteten Realwelt-Umgebung wahrnimmt.

12. Trainingssystem nach Anspruch 9, wobei ein erster Dritter der mindestens zwei Dritten topografische Daten speichert, welche die Realwelt-Trainingsroute betreffen, wobei ein zweiter Dritter der mindestens zwei Dritten Video- oder statische Bilder der Realwelt-Trainingsroute speichert, und wobei das ferne Kommunikationssystem die Video- oder statischen Bilder der Anzeigeprogrammierung mit Steuersignalen synchronisiert, welche die topografischen Daten repräsentieren, sodass eine zeitliche Beziehung zwischen Änderungen in den Steuersignalen und der Anzeigeprogrammierung besteht.

13. Trainingssystem nach Anspruch 1, das weiter mehrere Dritte in Kommunikation mit dem Netz umfasst, wobei die mehreren Dritten Daten speichern, welche ein oder mehrere Merkmale der Realwelt-Trainingsroute betreffen, wobei die mehreren Dritten (i) eine oder mehrere Datenbasen, die die Realwelt-Trainingsroute betreffende topografische Daten speichern, und (ii) eine oder mehrere Datenbasen, die die Realwelt-Trainingsroute betreffende Bilddaten speichern, umfassen,
wobei die Trainingsvorrichtung für eine Kommunikation mit dem Netz eingerichtet ist, wobei die Trainingsvorrichtung die Trainingsprogrammierung empfangen kann, die eingerichtet ist, um den einen oder die mehreren Betriebsparameter der Trainingsvorrichtung zu steuern, um die Realwelt-Trainingsroute im Wesentlichen zu simulieren; und
wobei das ferne Kommunikationssystem außerhalb der mehreren Dritten und fern von den mehreren Dritten ist, wobei das ferne Kommunikationssystem eingerichtet ist, um es einem Benutzer der Trainingsvorrichtung zu ermöglichen, die Realwelt-Trainingsroute zu definieren, wobei das ferne Kommunikationssystem eingerichtet ist, um mit den mehreren Dritten über das Netz zu kommunizieren, um die das eine oder die mehreren Merkmale der Realwelt-Trainingsroute betreffenden Daten abzurufen, wobei das ferne Kommunikationssystem eingerichtet ist, um die von den mehreren Datenbasen abgerufenen Daten zu verwenden, um die Trainingsprogrammierung zu erzeugen, wobei die Erzeugung der Trainingsprogrammierung umfasst: (i) ein Verwenden der Bilddaten, um eine Anzeigeprogrammierung zu erzeugen, wobei die Bilddaten mehrere sequentielle statische Bilder der Realwelt-Trainingsroute umfassen, welche auf der Trainingsvorrichtungsanzeige angezeigt werden können; (ii) ein Verwenden der topografischen Daten, um Steuersignale zu erzeugen, wobei die Steuersignale eingerichtet sind, um den einen oder die mehreren Betriebsparameter der Trainingsvorrichtung einzustellen, um das Gelände der Realwelt-Trainingsroute im Wesentlichen zu simulieren; und (iii) ein Synchronisieren der mehreren sequentiellen statischen Bilder der Anzeigeprogrammierung mit den Steuersignalen, wobei die Synchronisation der Steuersignale und der mehreren sequentiellen statischen Bilder es dem Benutzer der Trainingsvorrichtung ermöglicht, das Gelände der Realwelt-Trainingsroute auf der Trainingsvorrichtung im Wesentlichen wahrzunehmen, während er gleichzeitig Bilder der dem simulierten Gelände zugeordneten Realwelt-Trainingsroute betrachtet, und wobei das ferne Kommunikationssystem eingerichtet ist, um die Trainingsprogrammierung zu der Trainingsvorrichtung zu übermitteln.

14. Verfahren zum Erzeugen einer Trainingsprogrammierung, um eine ferne Realwelt-Trainingsroute auf einer Trainingsvorrichtung im Wesentlichen zu simulieren, das umfasst:
Empfangen, durch ein fernes Kommunikationssystem (14), eines oder mehrerer Parametern zum Definieren der fernen Realwelt-Trainingsroute;
Abrufen, durch das ferne Kommunikationssystem, einer ersten Art von Daten, die die ferne Realwelt-Trainingsroute betreffen, von einer ersten externen Drittdatenbasis der mindestens zwei externen Drittdatenbasen;
Abrufen, durch das ferne Kommunikationssystem, einer zweiten Art von Daten, die die ferne Realwelt-Trainingsroute betreffen, von einer zweiten externen Drittdatenbasis der mindestens zwei externen Drittdatenbasen;
Erzeugen, durch das ferne Kommunikationssystem, eines oder mehrerer Steuersignale unter Verwendung der ersten Art von Daten, welche von der ersten externen Drittdatenbasis abgerufen wurden, wobei die Steuersignale eingerichtet sind, um die Trainingsvorrichtung zu veranlassen, topografische Merkmale der fernen Realwelt-Trainingsroute im Wesentlichen zu simulieren;
Erzeugen, durch das ferne Kommunikationssystem, einer Anzeigeprogrammierung unter Verwendung der zweiten Art von Daten, welche von der zweiten externen Drittdatenbasis abgerufen wurden, wobei die Anzeigeprogrammierung Bilder der fernen Realwelt-Trainingsroute umfasst; und
Synchronisieren, durch das ferne Kommunikationssystem, des einen oder der mehreren Steuersignale und der Anzeigeprogrammierung.

15. Verfahren nach Anspruch 14, wobei die erste Art von Daten topografische Daten umfasst und die zweite Art von Daten Bilddaten umfasst, und wobei das Synchronisieren des einen oder der mehreren Steuersignale und der Anzeigeprogrammierung ein zeitliches Korrelieren von Änderungen des einen oder der mehreren Steuersignale mit Änderungen in der Anzeigeprogrammierung umfasst.

## Revendications

1. Système d'exercice configuré pour simuler un parcours d'exercice du monde réel, le système d'exercice comprenant :
un dispositif d'exercice (12) comprenant un élément mobile pour un déplacement lors de l'exécution d'un exercice par un utilisateur, au moins un actionneur pour commander un ou plusieurs paramètres de fonctionnement du dispositif d'exercice, et un afficheur, le dispositif d'exercice étant conçu pour recevoir une programmation d'exercice et utiliser la programmation d'exercice pour simuler sensiblement un ou plusieurs aspects du parcours d'exercice du monde réel, la programmation d'exercice comprenant un ou plusieurs signaux de commande représentatifs de changements à apporter auxdits un ou plusieurs paramètres de fonctionnement pour simuler sensiblement le parcours d'exercice du monde réel ;
un système de communication à distance (14) conçu pour utiliser des données relatives au parcours d'exercice du monde réel pour générer la programmation d'exercice,
dans lequel le système de communication à distance est configuré pour récupérer les données, les données étant mémorisées à l'extérieur du système de communication à distance,
dans lequel le système de communication à distance est conçu pour générer une programmation d'affichage pour une incorporation dans la programmation d'exercice, la programmation d'affichage comprenant des données d'image récupérées dans au moins une base de données à l'extérieur du système de communication, dans lequel la programmation d'affichage est affichée sur l'afficheur du dispositif d'exercice,
dans lequel le système de communication à distance est configuré pour synchroniser les données et les signaux de commande pour créer la programmation d'exercice pour simuler sensiblement le parcours d'exercice du monde réel ; et
un réseau (18) conçu pour faciliter la communication de la programmation d'exercice du système de communication à distance au dispositif d'exercice.

2. Système d'exercice selon la revendication 1, dans lequel les données d'image comprennent une pluralité d'images statiques séquentielles du parcours d'exercice du monde réel, et/ou dans lequel les données d'image comprennent des images vidéo du parcours d'exercice du monde réel.

3. Système d'exercice selon la revendication 1, dans lequel les signaux de commande commandent lesdits un ou plusieurs paramètres de fonctionnement alors que l'utilisateur regarde les images du parcours d'exercice du monde réel fournies par la programmation d'affichage sur l'afficheur du dispositif d'exercice.

4. Système d'exercice selon la revendication 1, dans lequel le système de communication à distance synchronise les signaux de commande et la programmation d'affichage de sorte qu'il y ait une relation temporelle entre les changements des signaux de commande et les images du parcours d'exercice du monde réel fournies par la programmation d'affichage, dans lequel la synchronisation de la programmation d'affichage et des signaux de commande fournit à l'utilisateur la capacité de regarder les images du parcours d'exercice du monde réel tout en ressentant sensiblement, au moyen desdits un ou plusieurs paramètres de fonctionnement changeants, les caractéristiques topographiques de l'environnement de monde réel vu dans les images de la programmation d'affichage.

5. Système d'exercice selon la revendication 1, dans lequel les données utilisées pour générer la programmation d'exercice comprennent des données de carte, des données topographiques, des données vidéo, des données d'image, ou une combinaison de celles-ci.

6. Système d'exercice selon la revendication 1, dans lequel le système de communication à distance est conçu pour communiquer avec au moins deux tiers pour obtenir les données utilisées pour générer la programmation d'exercice.

7. Système d'exercice selon la revendication 6, dans lequel un premier tiers desdits au moins deux tiers mémorise les données topographiques relatives au parcours d'exercice du monde réel, un deuxième tiers desdits au moins deux tiers mémorise les images vidéo ou statiques du parcours d'exercice du monde réel, et le système de communication à distance synchronise les images vidéo ou statiques avec les signaux de commande représentatifs des données topographiques.

8. Système d'exercice selon la revendication 1, dans lequel le système de communication à distance est conçu pour communiquer avec un utilisateur du dispositif d'exercice pour permettre à l'utilisateur de sélectionner un ou plusieurs paramètres définis par l'utilisateur du parcours d'exercice du monde réel, dans lequel lesdits un ou plusieurs paramètres sélectionnables par l'utilisateur du parcours d'exercice du monde réel comprennent un point de début, un point de fin, une distance totale, un changement d'élévation total, ou une combinaison de ceux-ci.

9. Système d'exercice selon la revendication 1, dans lequel le dispositif d'exercice comprend une interface utilisateur qui permet à un utilisateur de définir le parcours d'exercice du monde réel en sélectionnant un ou plusieurs paramètres du parcours d'exercice du monde réel ; et
dans lequel le système de communication à distance est conçu pour une communication avec le dispositif d'exercice, dans lequel le système de communication à distance est conçu pour recevoir lesdits un ou plusieurs paramètres définis par l'utilisateur du parcours d'exercice du monde réel et communiquer avec au moins deux tiers pour récupérer les données mémorisées par lesdits au moins deux tiers, relatives au parcours d'exercice du monde réel, et dans lequel le système de communication à distance est conçu pour utiliser les données récupérées auprès desdits au moins deux tiers pour générer la programmation d'exercice qui comprend des signaux de commande pour commander le fonctionnement du dispositif d'exercice et la programmation d'affichage synchronisée avec les signaux de commande.

10. Système d'exercice selon la revendication 9, dans lequel le système de communication à distance communique avec lesdits au moins deux tiers par l'intermédiaire d'un réseau, lesdits au moins deux tiers comprenant au moins deux bases de données externes et à distance du système de communication à distance, dans lequel lesdites au moins deux bases de données sont accessibles par l'intermédiaire d'un ou de plusieurs sites Web.

11. Système d'exercice selon la revendication 9, dans lequel le système de communication à distance utilise les données topographiques mémorisées par l'un desdits au moins deux tiers pour générer les signaux de commande qui sont configurés pour ajuster un ou plusieurs paramètres de fonctionnement du dispositif d'exercice, dans lequel le système de communication à distance utilise les données d'image mémorisées par un autre desdits au moins deux tiers pour générer la programmation d'affichage, dans lequel les données d'image comprennent une pluralité d'images statiques séquentielles du parcours d'exercice du monde réel, dans lequel la synchronisation de la programmation d'affichage et des signaux de commande fournit à l'utilisateur la capacité de voir les images du parcours d'exercice du monde réel tout en ressentant sensiblement, au moyen desdits un ou plusieurs paramètres de fonctionnement changeants, les caractéristiques topographiques de l'environnement de monde réel vu dans les images de la programmation d'affichage.

12. Système d'exercice selon la revendication 9, dans lequel un premier tiers desdits au moins deux tiers mémorise les données topographiques relatives au parcours d'exercice du monde réel, un deuxième tiers desdits au moins deux tiers mémorise les images vidéo ou statiques du parcours d'exercice du monde réel, et le système de communication à distance synchronise les images vidéo ou statiques de la programmation d'affichage avec les signaux de commande représentatifs des données topographiques de sorte qu'il y ait une relation temporelle entre les changements des signaux de commande et la programmation d'affichage.

13. Système d'exercice selon la revendication 1, comprenant en outre une pluralité de tiers en communication avec le réseau, la pluralité de tiers mémorisant des données relatives à une ou plusieurs caractéristiques du parcours d'exercice du monde réel, la pluralité de tiers comprenant (i) une ou plusieurs bases de données qui mémorisent des données topographiques relatives au parcours d'exercice du monde réel, et (ii) une ou plusieurs autres bases de données qui mémorisent des données d'image relatives au parcours d'exercice du monde réel,
dans lequel le dispositif d'exercice est conçu pour une communication avec le réseau, le dispositif d'exercice étant sensible à la programmation d'exercice qui est conçue pour commander lesdits un ou plusieurs paramètres de fonctionnement du dispositif d'exercice pour simuler sensiblement le parcours d'exercice du monde réel ; et
dans lequel le système de communication à distance est à l'extérieur et à distance de la pluralité de tiers, le système de communication à distance étant conçu pour permettre à un utilisateur du dispositif d'exercice de définir le parcours d'exercice du monde réel, le système de communication à distance étant conçu pour communiquer avec la pluralité de tiers par l'intermédiaire du réseau pour récupérer les données relatives auxdites une ou plusieurs caractéristiques du parcours d'exercice du monde réel, le système de communication à distance étant conçu pour utiliser les données récupérées dans la pluralité de bases de données pour générer la programmation d'exercice, dans lequel la génération de la programmation d'exercice comprend (i) l'utilisation des données d'image pour générer la programmation d'affichage, les données d'image comprenant une pluralité d'images statiques séquentielles du parcours d'exercice du monde réel qui peuvent être affichées sur l'afficheur du dispositif d'exercice ; (ii) l'utilisation des données topographiques pour générer des signaux de commande, les signaux de commande étant conçus pour commander lesdits un ou plusieurs paramètres de fonctionnement du dispositif d'exercice pour simuler sensiblement le terrain du parcours d'exercice du monde réel ; et (iii) la synchronisation de la pluralité d'images statiques séquentielles de la programmation d'affichage avec les signaux de commande, la synchronisation des signaux de commande et de la pluralité d'images statiques séquentielles permettant à l'utilisateur du dispositif d'exercice de ressentir sensiblement le terrain du parcours d'exercice du monde réel sur le dispositif d'exercice tout en regardant simultanément les images du parcours d'exercice du monde réel associées au terrain simulé, et dans lequel le système de communication à distance est conçu pour mettre en communication la programmation d'exercice et le dispositif d'exercice.

14. Procédé pour générer une programmation d'exercice pour simuler sensiblement un parcours d'exercice du monde réel à distance sur un dispositif d'exercice, comprenant :
la réception, par un système de communication à distance (14), d'un ou de plusieurs paramètres pour définir le parcours d'exercice du monde réel à distance ;
la récupération, par le système de communication à distance, d'un premier type de données relatives au parcours d'exercice du monde réel à distance dans une première base de données externe de tiers desdites au moins deux bases de données externes de tiers ;
la récupération, par le système de communication à distance, d'un deuxième type de données relatives au parcours d'exercice du monde réel à distance dans une deuxième base de données externe de tiers desdites aux moins deux bases de données externes de tiers ;
la génération, par le système de communication à distance, d'un ou de plusieurs signaux de commande en utilisant le premier type de données récupéré dans la première base de données externe de tiers, les signaux de commande étant conçus pour amener le dispositif d'exercice à simuler sensiblement les caractéristiques topographiques du parcours d'exercice du monde réel à distance ;
la génération, par le système de communication à distance, d'une programmation d'affichage en utilisant le deuxième type de données récupéré dans la deuxième base de données externe de tiers, la programmation d'affichage comprenant des images du parcours d'exercice du monde réel à distance ; et
la synchronisation, par le système de communication à distance, desdits un ou plusieurs signaux de commande et de la programmation d'affichage.

15. Procédé selon la revendication 14, dans lequel le premier type de données comprend des données topographiques et le deuxième type de données comprend des données d'image, et dans lequel la synchronisation desdits un ou plusieurs signaux de commande et de la programmation d'affichage comprend la corrélation temporelle des changements desdits un ou plusieurs signaux de commande avec les changements de la programmation d'affichage.
